# EUROPEAN PATENT APPLICATION

(11) **EP 3 666 752 A1**
(43) Date of publication of application: **17.06.2020**
(21) Application number: 18212487.5
(22) Date of filing: 13.12.2018
(51) Int. Cl.: C07C 239/08, C07C 323/36, C07D 213/24

(54) **TRACELESS REDUCTIVELY CLEAVABLE LINKER MOLECULES FOR PEPTIDE PURIFICATION**

(71) Applicant: Belyntic GmbH, 12489 Berlin (DE)
(72) Inventor: ZITTERBART, Robert, 13158 Berlin (DE); REIMANN, Oliver, 10117 Berlin (DE)
(74) Representative: Schulz Junghans Patentanwälte PartGmbB

(57) **Abstract**

The present invention relates to linker molecules of formula (1), X-T_{b}-Vₐ-U-Y-Z (1) and a method for purifying peptides using said linker molecules. The linker molecule can be coupled to a purification resin via the moiety X and to a peptide via the moiety Y under the release of the leaving group Z. T is an optional spacer moiety and V is an optional electron withdrawing moiety. U is an aryl or 5- or 6-membered heteroaryl moiety bound to at least one electron withdrawing moiety V, W or E. The linker is stable under acidic conditions and releases the peptide upon addition of a reducing agent.

SEQUENCE LISTING

<110>  Belyntic GmbH

<120>  Traceless Reductively Cleavable Linker molecules for Peptide
       Purification

<130>  bey102ep-1

<160>  6

<170>  PatentIn version 3.5

<210>  1
<211>  15
<212>  PRT
<213>  Artificial Sequence

<220>
<223>  fragment 2-16 of the Histone H3 protein

<400>  1

Ala Arg Thr Lys Gln Thr Ala Arg Lys Ser Thr Gly Gly Lys Ala
1               5                   10                  15


<210>  2
<211>  13
<212>  PRT
<213>  Artificial Sequence

<220>
<223>  test peptide

<400>  2

Ala Lys Ala Asp Glu Val Ser Leu His Lys Trp Tyr Gly
1               5                   10


<210>  3
<211>  15
<212>  PRT
<213>  Artificial Sequence

<220>
<223>  fragment 81-95 of the human cytomegalovirus lower matrix
       phosphoprotein (CMV)

<400>  3

Tyr Phe Thr Gly Ser Glu Val Glu Asn Val Ser Val Asn Val His
1               5                   10                  15


<210>  4
<211>  11
<212>  PRT
<213>  Artificial Sequence

<220>
<223>  fragment 510-520 of humane Lemur Tyrosine Kinase 3 (LMTK3)

<400>  4

Pro Ser Asn Pro Phe Tyr Glu Ala Leu Ser Thr
1               5                   10

<210>  5
<211>  20
<212>  PRT
<213>  Artificial Sequence

<220>
<223>  fragment 1-20 of humane amyloid beta

<400>  5

Asp Ala Glu Phe Arg His Asp Ser Gly Tyr Glu Val His His Gln Lys
1               5                   10                  15

Leu Val Phe Phe
            20

<210>  6
<211>  13
<212>  PRT
<213>  Artificial Sequence

<220>
<223>  test peptide

<400>  6

Cys Lys Ala Asp Glu Val Ser Met His Lys Trp Tyr Gly
1               5                   10

## Description

### Field of the invention

The present invention relates to a method of purifying peptides or peptide nucleic acids produced by solid phase peptide synthesis (SPPS) and linker molecules for use in said purification.

### Background of the invention

Solid phase peptide synthesis is an established method for the synthesis of peptides. A standard procedure is the coupling of the first N-terminally protected amino acid to a synthesis resin followed by repeated cycles of deprotection of the N-terminus, coupling the next N-terminally protected amino acid and capping of unreacted peptide sequences. Finally, the synthesized peptide is cleaved off the synthesis resin and purified.

A widely-used method for the purification of peptides is the preparative high performance liquid chromatography (HPLC). Disadvantageous at this method is the poor scalability with regard to the desired production quantities, so that different quantities cannot be produced with one and the same system. This causes relatively high acquisition costs for the corresponding complex devices. A further disadvantage is the requirement of relatively extensive knowledge for a correct analytical assessment of the individual fractions. Additionally, HPLC purification involves a high consumption of solvents and occasionally of column material (solid phase) during operation.

Therefore, methods that are cheaper and less prone to faults would be advantageous for reducing the costs of peptide production.

Alternative methods use linker molecules that can be attached to peptides and thereafter coupled to a functionalized solid phase that is used during purification.

EP 0 552 368 A1 describes a linker molecule bearing a thiol that can be covalently bound to a purification support. However, the method is not suitable for thiol-containing peptides such as those comprising the amino acid cysteine or penicillamine.

EP 2 501 711 B1 proposes an analogous method in which the linker is bound to a solid phase via a 1,3-dipolar cycloaddition between an azide (-N₃) and an alkyne which requires the presence of copper. However, peptides that comprise methionine, cysteine; arginine or lysine may complex copper making a removal difficult. Due to the toxicity of copper, such peptides are not suitable for all applications such as pharmaceutical use.

WO2017129818 (A1) discloses linker molecules that may be coupled to a peptide that is still bound to the synthesis resin after SPPS. The peptide is then cleaved off the synthesis by applying commonly used TFA conditions. However, a disadvantage of the linker molecules that form a benzyl-carbamate with the peptide as disclosed in WO2017129818 (A1) is their lability to acidic treatment (TFA > 50%, in presence of water pH < 0). Premature decay of the linker molecules causes significantly reduced yield of purified peptide.

Unwanted side reactions also occur with peptides that contain Thr, Ser or Cys at their N-terminus due to a nucleophilic attack of the β-hydroxyl or β-thiol group on the sulfo-ethyleneyl carbamate moiety of the linker under the basic conditions (pH > 9) that are used for release of the peptide. Further side reactions of linker molecules disclosed in WO2017129818 (A1) are aspartimide formation and the conversion of arginine to citrulline at Arg-Glu sequences and disulfide formation and nucleophilic side reactions by internal Cys residues under basic conditions.

Furthermore, the sulfone linkers described in WO2017129818 (A1) suffer from a reactive vinyl sulfone moiety that remains on the solid report and needs an additional quenching step.

To overcome the disadvantage of side reactions under basic conditions and premature decay of the linker under acidic conditions, the present invention provides linker molecules that are stable under TFA conditions and allow peptide release under mild acidic conditions, in particular at pH ≤ 7.

### Description

According to a first aspect of the invention, a compound of formula 1, X-T_{b}-Vₐ-U-Y-Z (1), is provided, wherein
- X is selected from a moiety of formula 2, 2a, 3, 3a or 4, in particular of formula 2, 2a, 3 or 3a, more particularly of formula 2 or 2a, 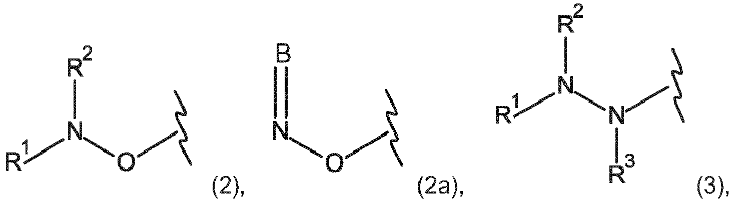 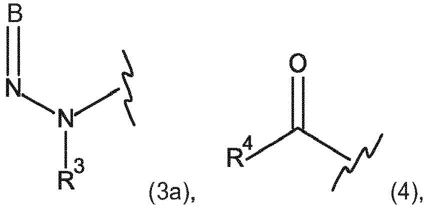 wherein
   - each R¹ and R² is independently from each other selected from H or B, wherein at least R¹ or R² is B,
   - R³ is selected from H or B,
   - R⁴ is selected from H, C₁-C₁₂-alkyl or aryl, wherein the aldehyde or keto group may be protected by an acid labile protecting group,
   - B is an acid labile amine protecting group,
- T is a linear or branched spacer comprising at least one of the moieties -C₁₋₁₂alkyl-, (-C₂H₄O-)₁₋₁₂, -C(=O)-, -C(=O)-JR⁹-, -JR⁹-C(=O)-, -JR⁹-, wherein
   J is C or N, in particular N,
   in particular T is a spacer selected from
   -C₁-C₁₂-alkyl-, in particular -C₁₋₆-alkyl-, more particularly -C₁₋₃-alkyl-, -R⁵-C(=O)-, -R⁵-C(=O)-NR⁹-R⁶-, -R⁵-C(=O)-NR⁹-, -C(=O)-NR⁹-R⁶-, -R⁵-NR⁹-C(=O)-R⁶-, -R⁵-NR⁹-R^{5'}-NR^{9'}-C(=O)-R⁶-, - R⁵-C(=O)-NR⁹-R^{5'}-NR^{9'}-C(=O)-R⁶-, -R⁵-NR⁹-, -R⁵-NR⁹-R⁶-, -R⁵-NR⁹-R^{5'}-NR^{9'}-R⁶-, -R⁵-C(=O)-NR⁹-R^{5'}-NR^{9'}-R⁶-, -R⁵-C(=O)-O-R⁶-,-C(=O)-O-R⁶-, -R⁵-phenyl-R⁶-, -R⁵-phenyl-, -phenyl-R⁶-, -phenyl-, wherein
   R⁵, R^{5'} and R⁶ are independently from each other selected from C₁-C₁₂-alkyl or (-C₂H₄O-)₁₋₁₂, in particular C₁-C₆ alkyl, particularly C₁-C₃ alkyl, and wherein
   R⁹ and R^{9'} are independently from each other selected from H, C₁₋₄-alkyl, -C₁₋₆-alkyl-NH₂, -C₁₋₆-alkyl-NHB, -C₁₋₆-alkyl-NB₂, -R¹⁵, -C₁₋₆-alkyl-R¹⁵, -C₁₋₆-alkyl-NH-R¹⁵, in particular from H and C₁₋₂-alkyl, more particularly R⁹ is H, wherein
      B is an independently selected acid labile amine protecting group,
      R¹⁵ is a blocking agent that is able to react with an aldehyde moiety, in particular R¹⁵ is selected from cysteinyl, threoninyl, 2-mercaptoethanol, cysteamine, ethandithiole, hydroxylamine, O-methylhydroxylamine, N-methylhydroxylamine, dithiothreitol, hydrazine, in particular cysteinyl and N-methylhydroxylamine, more particularly cysteinyl, wherein amine and/or thiol moieties of the blocking agent may be protected by an independently selected acid labile amine protecting group B, particularly Boc, and/or an acid labile thiol protecting group, particularly trityl.
- b is 0 or 1, in particular 1,
- V is an electron-withdrawing moiety selected from -NR¹¹-(=O)-, -C(=O)-NR¹¹-,-S(=O)-, -NR¹²-, -piperazinyl-, -pyridinyl, pyrimidinyl, pyrazinyl, pyridazinyl, 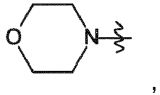 -C(=O)-, -C(=O)-O-, in particular -NR¹¹-(=O)-, -C(=O)-NR¹¹-, -S(=O)-, -NR¹²-, -piperazinyl-, -pyridinyl-, pyrimidinyl, more particularly from -NH-C(=O)-, -C(=O)-NH-, -N-(CH₃)-, -piperazinyl-, -pyridinyl-, pyrimidinyl, wherein
   R¹¹ is selected from H and C₁₋₄-alkyl, in particular from H and C₁₋₂-alkyl, more particularly R¹¹ is H,
   R¹² is selected from H and C₁₋₄-alkyl, in particular from H and C₁₋₂-alkyl, more particularly R¹² is methyl,
- a is 0 or 1, wherein the sum of a and b is 1 or 2,
- U is a phenyl or a five- or six-membered heteroaryl moiety, in particular a phenyl or a six-membered heteroaryl moiety, more particularly a phenyl, that is bound to at least one of the moieties V, W_{q} and Eₙ and that may optionally be substituted by C₁₋₆-alkyl, in particular C₁₋₃-alkyl, wherein
   V is defined as described above,
   W is selected from -N₃, -S(=O)-R⁸, -S-S-R⁸, -O-CH₂-N₃, -O-C(=O)-O-CH₂-N₃,-N=N-phenyl, -N=N-R⁸, 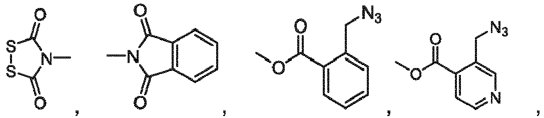 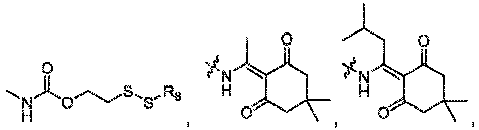 in particular -N₃, -N=N-R⁸, -O-CH₂-N₃, -S-S-R⁸, wherein
      R⁸ is pyridyl, pyrimidinyl, pyrazinyl, pyridazyl, -C₁-C₆-alkyl or -(CH₂)ₚ-NMe₂, in particular pyridyl or -C₁-C₆-alkyl, with p being 1, 2, 3 or 4,
   E is an electron withdrawing group under acidic conditions,
   n being is an integer between 0 and 4, in particular 0 and 2, more particularly 0 or 1, and q is an integer between 0 and 4, in particular 0 and 2, more particularly 0 and 1, wherein the sum of n and q is equal or lower than 4, and wherein
   in case of U being a phenyl moiety and Y being -(CH₂)ₘ-O-C(=O)-, the sum of Hammett constants of V, W, E under acidic conditions is larger than 0.45,
   - Y is -(CH₂)ₘ-C(=O)- or -(CH₂)ₘ-O-C(=O)- with m being 1, 2 or 3, in particular 1 or 2, more particularly 1,
   - Z is an electron-withdrawing leaving group.

In certain embodiments,
- X is selected from a moiety of formula 2, 2a, 3, 3a or 4, in particular of formula 2, 2a, 3 or 3a, more particularly of formula 2 or 2a, 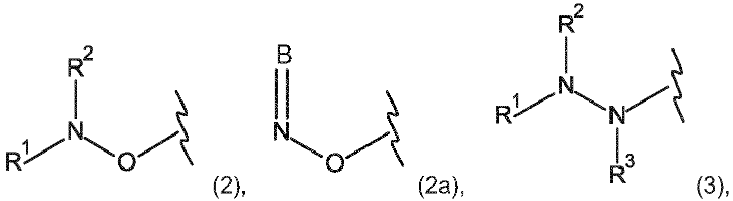 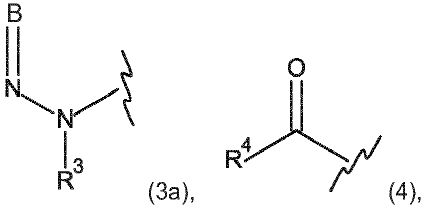 wherein
   - each R¹ and R² is independently from each other selected from H or B, wherein at least R¹ or R² is B,
   - R³ is selected from H or B,
   - R⁴ is selected from H, C₁-C₁₂-alkyl or aryl, wherein the aldehyde or keto group may be protected by an acid labile protecting group,
   - B is an acid labile amine protecting group,
- T is a spacer selected from
   -C₁-C₁₂-alkyl-, in particular C₁₋₆-alkyl, more particularly C₁₋₃-alkyl, -R⁵-C(=O)-,-R⁵-C(=O)-NR⁹-R-, -R⁵-C(=O)-NR⁹-, -C(=O)-NR⁹-R⁶-, -R⁵-NR⁹-C(=O)-R⁶-, -R⁵-NR⁹-R^{5'}-NR^{9'}C(=O)-R⁶-, -R⁵-C(=O)-NR⁹-R^{5'}-NR^{9'}-C(=O)-R⁶-, -R⁵-C(=O)-O-R⁶-,-C(=O)-O-R⁶-, -R⁵-phenyl-R⁶-, -R⁵-phenyl-, -phenyl-R⁶-, -phenyl-, wherein
   R⁵, R^{5'} and R⁶ are independently from each other selected from C₁-C₁₂-alkyl, in particular C₁-C₆ alkyl, particularly C₁-C₃ alkyl, and wherein R⁹ and R^{9'} are independently from each other selected from H and C₁₋₄-alkyl, in particular from H and C₁₋₂-alkyl, more particularly R⁹ is H,
- b is 0 or 1, in particular 1,
- V is an electron-withdrawing moiety selected from -NR¹¹-(=O)-, -C(=O)-NR¹¹-,-S(=O)-, -NR¹²-, -piperazinyl-, -pyridinyl, pyrimidinyl, pyrazinyl, pyridazinyl, 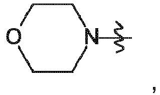 -C(=O)-, -C(=O)-O-, in particular -NR¹¹-(=O)-, -C(=O)-NR¹¹-, S(=O)-, -NR¹²-, -piperazinyl-, -pyridinyl-, pyrimidinyl, more particularly from -NH-C(=O)-,-C(=O)-NH-, -N-(CH₃)-, -piperazinyl-, -pyridinyl-, pyrimidinyl, wherein
   R¹¹ is selected from H and C₁₋₄-alkyl, in particular from H and C₁₋₂-alkyl, more particularly R¹¹ is H,
   R¹² is selected from H and C₁₋₄-alkyl, in particular from H and C₁₋₂-alkyl, more particularly R¹² is methyl,
- a is 0 or 1, wherein the sum of a and b is 1 or 2,
- U is a phenyl or a five- or six-membered heteroaryl moiety, in particular a phenyl or a six-membered heteroaryl moiety, more particularly a phenyl, that is bound to at least one of the moieties V, W_{q} and Eₙ and that may optionally be substituted by C₁₋₆-alkyl, in particular C₁₋₃-alkyl, wherein
   V is defined as described above,
   W is selected from -N₃, -S(=O)-R⁸, -S-S-R⁸, -O-CH₂-N₃, -O-C(=O)-O-CH₂-N₃,-N=N-phenyl, -N=N-R⁸, 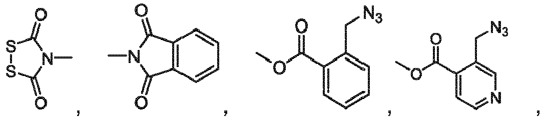 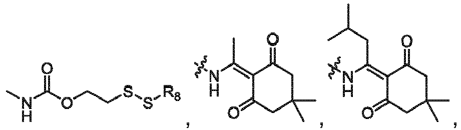 in particular -N₃, -N=N-R⁸, -O-CH₂-N₃, -S-S-R⁸, wherein
      R⁸ is pyridyl, pyrimidinyl, pyrazinyl, pyridazyl, -C₁-C₆-alkyl or -(CH₂)ₚ-NMe₂, in particular pyridyl or -C₁-C₆-alkyl, with p being 1, 2, 3 or 4,
   E is an electron withdrawing group under acidic conditions,
   n being is an integer between 0 and 4, in particular 0 and 2, more particularly 0 or 1, and q is an integer between 0 and 4, in particular 0 and 2, more particularly 0 and 1, wherein the sum of n and q is equal or lower than 4, and wherein
   in case of U being a phenyl moiety and Y being -(CH₂)ₘ-O-C(=O)-, the sum of Hammett constants of V, W, E under acidic conditions is larger than 0.45,
- Y is -(CH₂)ₘ-C(=O)- or -(CH₂)ₘ-O-C(=O)- with m being 1, 2 or 3, in particular 1 or 2, more particularly 1,
- Z is an electron-withdrawing leaving group.

In certain embodiments,
- X is selected from a moiety of formula 2, 2a, 3, 3a or 4, in particular of formula 2, 2a, 3 or 3a, more particularly of formula 2 or 2a, most particularly from 2, 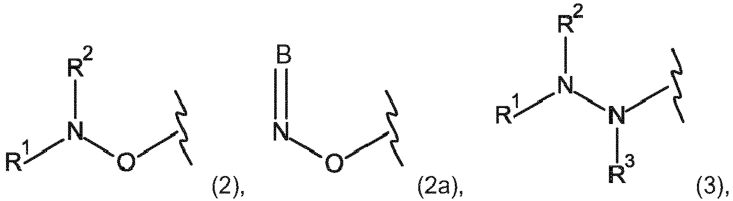 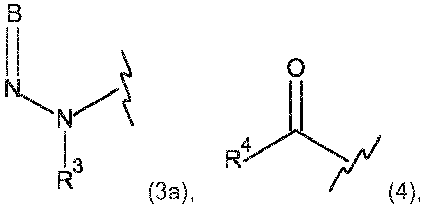 wherein
   - each R¹ and R² is independently from each other selected from H or B, wherein at least R¹ or R² is B,
   - R³ is selected from H or B,
   - R⁴ is selected from H, C₁-C₁₂-alkyl or aryl, wherein the aldehyde or keto group may be protected by an acid labile protecting group,
   - B is an acid labile amine protecting group,
- T is a spacer selected from
   -C₁-C₁₂-alkyl-, in particular C₁₋₆-alkyl, more particularly C₁₋₃-alkyl, -R⁵-C(=O)-NR⁹-R⁶-, -C(=O)-NR⁹-R⁶-, -R⁵-NR⁹-R⁵-NR⁹C(=O)-R⁶-, -R⁵-NR⁹-C(=O)-R⁶-, -R⁵-C(=O)-O-R⁶-, -C(=O)-O-R⁶-, -R⁵-phenyl-R⁶-, -R⁵-phenyl-, -phenyl-R⁶-, -phenyl-, wherein
   R⁵ and R⁶ are independently from each other selected from C₁-C₁₂-alkyl, in particular C₁-C₆ alkyl, particularly C₁-C₃ alkyl, and wherein R⁹ is selected from H and C₁₋₄-alkyl, in particular from H and C₁₋₂-alkyl, more particularly R⁹ is H,
- b is 0 or 1, in particular 1,
- V is an electron-withdrawing moiety selected from -NR¹¹-(=O)-, -C(=O)-NR¹¹-,-S(=O)-, -NR¹²-, -pyridinyl, pyrimidinyl, pyrazinyl, pyridazyl, -C(=O)-, -C(=O)-O-, in particular-NR¹¹-C(=O)-, S(=O)-, -NR¹²-, -pyridinyl-, pyrimidinyl, wherein
   R¹¹ is selected from H and C₁₋₄-alkyl, in particular from H and C₁₋₂-alkyl, more particularly R¹¹ is H,
   R¹² is selected from H and C₁₋₄-alkyl, in particular from H and C₁₋₂-alkyl, more particularly R¹² is methyl,
- a is 0 or 1, wherein the sum of a and b is 1 or 2,
- U is a phenyl or a five- or six-membered heteroaryl moiety, in particular a phenyl or a six-membered heteroaryl moiety, that is bound to at least one of the moieties V, W and E, wherein
   V is defined as described above,
   W is selected from -N₃, -S(=O)-R⁸, -SSR⁸, -OCH₂N₃, -OC(=O)OCH₂-N₃,
   -N=N-phenyl, -N=N-pyridine, 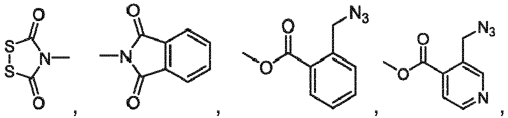 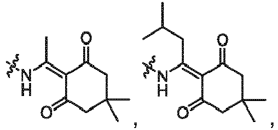 wherein
      R⁸ is pyridyl, pyrimidinyl, pyrazinyl, pyridazyl, -C₁-C₆-alkyl or -(CH₂)ₚ-NMe₂ with p being 1, 2, 3 or 4,
   E is selected from pyridyl, pyrimidinyl, pyrazinyl, pyridazyl, -F, -Cl, -Br, -I, -CN, -NO₂, -N₃, -CF₃, -SO₃H, -CO₂H, -C(=O)NH₂, -SO₂Me, -SOMe, -SO₂Et, -SOEt, in particular pyridyl, pyrimidinyl, pyrazinyl, pyridazyl, -F, -CI, -Br, -I, -CN, -NO₂, -N₃, -CF₃, -SO₃H, -CO₂H, with
      R¹³ being selected from -F, -Cl, -Br, -I, -PF₆, and wherein
   in case of U being a phenyl moiety and Y being -(CH₂)ₘ-O-C(=O)-, the sum of Hammett constants of V, W, E under acidic conditions is larger than 0.45,
- Y is -(CH₂)ₘ-C(=O)- or -(CH₂)ₘ-O-C(=O)- with m being 1, 2 or 3, in particular 1 or 2, more particularly 1,
- Z is an electron-withdrawing leaving group.

The linker molecule of formula 1 is suitable for the purification of peptides after solid phase peptide synthesis (SPPS).

A common approach for purifying peptides after SPPS using a linker molecule is coupling the linker molecule to the N-terminus of the peptide in a final coupling step. In this coupling step, the N-terminus of the peptide will nucleophilically attack the linker molecule to form a covalent carbamate or amide bond with the moiety Y of the linker while the electron withdrawing leaving group Z is released. Subsequently, the peptide-linker-construct is cleaved off the synthesis resin by addition of TFA.

Linkers according to the invention are stable under acidic conditions when the peptide-linker construct is cleaved off, e.g. by using TFA.

The moiety X of the linker may be coupled to a functionalized solid phase such as a resin that is used during purification. The moiety X may form a hydrazone bond or oxime bond by reacting with a suitable moiety of the functionalized solid phase such as aldehyde, ketone, amino-oxy or hydrazine.

The moiety T represents a spacer that is non-reactive under commonly applied purification conditions.

The moiety T is either directly bound to the moiety U or via the moiety V.

The moiety U contributes to the stability of the linker molecule under acidic conditions, particularly TFA > 50 %, in the presence of water pH < 0. This is either achieved by using a heterocyclic moiety or a phenyl moiety that is bound to at least one of the electron-withdrawing moieties E, W and V.

If electron-withdrawing moieties (E, W, V) are attached to the phenyl moiety, the benzylic position of the linker molecule linked to the peptide gets less electron density and is thus less susceptible to acid catalyzed degradation. For sufficient stability of the inventive linker molecule under acidic conditions, a certain threshold of electron-withdrawal has to be met. This threshold is expressed as the sum of Hammett constants (σₘ and σₚ) of V, W, E under acidic conditions being larger than 0.45. Hammett constants are calculated according to Hansch and Taft (1991), Chem. Rev. 91:165-195. A positive Hammett constant reflects the ability of the substituent to exert an electron withdrawing effect on the phenyl moiety and a negative value indicates that the substituent exerts an electron donating effect.

Hammett constants are empirically determined constants for substituents of the phenyl core in benzoic acid derivates in meta (σₘ) and para position (σₚ) resulting in different acidities (pKa). In the context of the present invention, the position is determined in relation to the binding of the moiety Y. For substituents in ortho position, Hammett values for the para position are a good approximation and therefore used in the context of the present invention to calculate the sum of Hammett values of the substituents V, W and E.

Of note, the Hammett constants are calculated for the substituents V, W and E under acidic conditions. For example, an amine moiety at neutral pH is characterized by the Hammett constants σₘ = -0.16 and σₚ = -0.66 and thus an electron-pushing substituent. Under acidic conditions, the amine moiety is protonated. For protonated amines, the Hammett constants are σₘ = +0.86 and σₚ = +0.60 indicating that protonated amines are electron-withdrawing substituents, this also includes aromatic amines which are able to withdraw electrons through a conjugated π-system from U in their protonated form whether directly as substituents on U or in π-conjugation of U.

The threshold of the sum of Hammet constants being larger than 0.45 applies for U being a phenyl moiety and Y being -(CH₂)ₘ-O-C(=O)- with m = 1, since the -O-C(=O)- comprise a good leaving group in the benzylic position, facilitating the acid catalyzed degradation. Therefore electron-density in the aromatic ring should be low enough to prevent stabilization of cations in the benzylic position in Y.

If the moiety Y is -(CH₂)ₘ-C(=O)-, this threshold is not necessary because -C(=O)- is not a good leaving group in the benzylic position.

For the stability of linker molecules under acidic conditions that comprise a heterocyclic moiety U, such extra threshold for the selection of specific moieties V, W and E is not necessary. As heterocyclic moieties *per se* are more electron-deficient compared to phenyl moieties, any combination of V, W and E appears to be sufficient for the stability of the linker molecule under acidic conditions. Especially when U is being a nitrogen containing heterocycle, the nitrogen will be protonated during acidic release of the peptide rendering the aromatic system of U especially low in electron density. Thus, not able to stabilize a benzylic cation.

The moiety Y is either a -C(=O)- or a -O-C(=O)- moiety. Upon coupling of the linker molecule, an amide (-C(=O)-NH-) or a carbamate (-O-C(=O)-NH-) moiety is formed between the linker molecule and the N-terminus of the peptide. After purification, the peptides are released from the linker molecule and thus the purification media under reductive conditions either by 1.4 or 1.6 elimination or nucleophilic attack. The reductive stimulus transforms W into its reduced version, now functioning as an electron donating group and a nucleophile, thus enabling the release of the peptide.

In certain embodiments, X is selected from a moiety of formula 2 or 3.

In certain embodiments, X is selected from a moiety of formula 2.

The reaction time required for the coupling of the moiety X to a functionalized solid phase by formation of a hydrazone or oxime bond is longer when a linker with a moiety of formula 4 is used and shorter when a linker with a moiety of formula 2 or 3 is used. The formation of the hydrazone bond between an aldehyde or ketone moiety of a solid support and a moiety X of formula 3 is reversible. Due to this reversibility, the inventors observed up to approximately 10 % loss of peptide material after each washing step during purification. In contrast to this, almost no loss of peptide material was observed when a linker with a moiety of formula 2 was used.

In certain embodiments, U is substituted by C₁₋₆-alkyl.

In certain embodiments, U is substituted by C₁₋₃-alkyl.

In certain embodiments, U is substituted by methyl.

If U is further substituted by one or more alkyl moieties, the Hammett values of the alkyl moieties are taken into consideration. In case of U being further substituted by a phenyl moiety or heteroatomic, the sum of Hammett values of V, W, E and the optional alkyl substituent is larger than 0.45.

In certain embodiments, E is selected from pyridyl, pyrimidinyl, pyrazinyl, pyridazyl, ,-N=N-phenyl, -N=N-R⁸, -F, -CI, -Br, -I, -CN, -NO₂, -N₃, -CF₃, -SO₃H, -CO₂H, -C(=O)NH₂, -SO₂Me,-SOMe, -SO₂Et, -SOEt with R⁸ being pyridyl, pyrimidinyl, pyrazinyl, pyridazyl, -C₁-C₆-alkyl or-(CH₂)ₚ-NMe₂, in particular pyridyl or -C₁-C₆-alkyl, with p being 1, 2, 3 or 4.

In certain embodiments, E is selected from pyridyl, pyrimidinyl, pyrazinyl, pyridazinyl,, -N=N-phenyl, -N=N-R⁸, -F, -CI, -Br, -I, -CN, -NO₂, -N₃, -CF₃, -SO₃H, -CO₂H with R⁸ being pyridyl, pyrimidinyl, pyrazinyl, pyridazyl, -C₁-C₆-alkyl or -(CH₂)ₚ-NMe₂, in particular pyridyl or -C₁-C₆-alkyl, with p being 1, 2, 3 or 4.

In certain embodiments, E is selected from pyridyl, pyrimidinyl, pyrazinyl, pyridazyl, -F, -Cl,-Br, -I, -CN, -NO₂, -N₃, -CF₃, -SO₃H, -CO₂H, -C(=O)NH₂, -SO₂Me, -SOMe, -SO₂Et, -SOEt.

In certain embodiments, E is selected from pyridyl, pyrimidinyl, pyrazinyl, pyridazinyl, -F, -CI, -Br, -I, -CN, -NO₂, -N₃, -CF₃, -SO₃H, -CO₂H.

In certain embodiments, E is selected pyridyl, pyrimidinyl, pyridazinyl or -Br.

In certain embodiments, B is selected from
- Boc (-C(=O)OtBu), Eei (=CMeOEt, 1-ethoxyethylidene), trityl (-C(Ph)₃), Mmt (-C(Ph)₂C₆H₄OMe), DMT (-C(Ph)(C₆H₄OMe)₂), Cbz (-C(=O)OCH₂Ph), benzylideneamine (=CPh), phtalimides (=(CO)₂C₆H₄), p-toluenesulfonamides (-SO₂C₆H₄Me), benzylamine (-CH₂Ph), acetamides (-COMe), trifluoroacetamide (-COCF₃), Dde (1-(4,4-dimethyl-2,6-dioxocyclohex-1-ylidene)-3-ethyl) and 1-(4,4-dimethyl-2,6-dioxocyclohex-1-ylidene)-3-methylbutyl (ivDde), wherein particularly B is Boc or Eei, wherein more particularly B is Boc, or the
- acetal- or ketal protecting groups are selected from 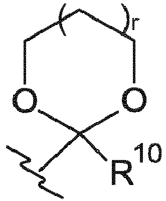 or 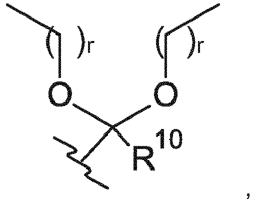 wherein r is 0 to 12, in particular 0 to 6, more particularly 0, 1 or 2, and
   R¹⁰ is a -C₁-C₁₂-alkyl-, in particular C₁₋₆-alkyl, more particularly C₁₋₃-alkyl.

In certain embodiments, B is selected from Boc (-C(=O)OtBu), Eei (=CMeOEt, 1-ethoxyethylidene), trityl (-C(Ph)₃), Mmt (-C(Ph)₂C₆H₄OMe), DMT (-C(Ph)(C₆H₄OMe)₂), Cbz (-C(=O)OCH₂Ph), benzylideneamine (=CPh), phtalimides (=(CO)₂C₆H₄), p-toluenesulfonamides (-SO₂C₆H₄Me), benzylamine (-CH₂Ph), acetamides (-COMe), trifluoroacetamide (-COCF₃), Dde (1-(4,4-dimethyl-2,6-dioxocyclohex-1-ylidene)-3-ethyl) and 1-(4,4-dimethyl-2,6-dioxocyclohex-1-ylidene)-3-methylbutyl (ivDde).

In certain embodiment, B is Boc or Eei (=CMeOEt, 1-ethoxyethylidene).

In certain embodiment, B is Boc.

In certain embodiments, B is selected from acetal- or ketal protecting groups are selected from 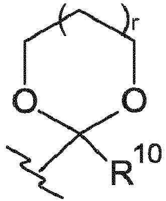 or 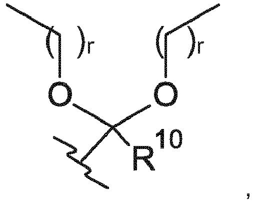 wherein r is 0 to 12, in particular 0 to 6, more particularly 0, 1 or 2, and R¹⁰ is -C₁-C₁₂-alkyl-, in particular C₁₋₆-alkyl.

In certain embodiments, T is a linear or branched spacer comprising at least one of the moieties -C₁₋₁₂-alkyl-, (-C₂H₄O-)₁₋₁₂, -C(=O)-, -C(=O)-JR⁹-, -JR⁹-C(=O)-, -JR⁹-, wherein J is C or N, in particular N, wherein R⁹ is independently selected from H, C₁₋₄-alkyl, -C₁₋₆-alkyl-NH₂,-C₁₋₆-alkyl-NHB, -C₁₋₆-alkyl-NB₂, in particular from H and C₁₋₂-alkyl, more particularly R⁹ is H, wherein B is an independently selected acid labile amine protecting group.

In certain embodiments, the total length of the spacer T is between 0.5 and 100 nm.

In certain embodiments, T is selected from -C₁-C₁₂-alkyl-,in particular C₁₋₆-alkyl, more particularly C₁₋₃-alkyl, -R⁵-C(=O)-, -R⁵-C(=O)-NR⁹-, -R⁵-NR⁹-C(=O)-R⁶-, -R⁵-C(=O)-NR⁹-R⁶-,-R⁵-C(=O)-NR⁹-R^{5'}-NR^{9'}C(=O)-R⁶-, in particular C₁₋₃-alkyl, -R⁵-C(=O)-NR⁹-, -R⁵-NR⁹-C(=O)-R⁶-, more particularly C₁₋₃-alkyl or -R⁵-C(=O)-NR⁹-, with R⁵, R^{5*}, R⁶, R^{9'} and R⁹ being as defined above.

In certain embodiments, T is a linear or branched spacer comprising at least one of the moieties -C₁₋₁₂alkyl-, -C(=O)-, -C(=O)-NR⁹-, -NR⁹-C(=O)-, -NR⁹-, wherein R⁹ is independently selected from H, C₁₋₄-alkyl, -C₁₋₆-alkyl-NH₂, -C₁₋₆-alkyl-NHB, -C₁₋₆-alkyl-NB₂, -R¹⁵, -C₁₋₆-alkyl-R¹⁵, -C₁₋₆-alkyl-NH-R¹⁵, in particular from H and C₁₋₂-alkyl, more particularly R⁹ is H, wherein B is an independently selected acid labile amine protecting group,

R¹⁵ is a blocking agent that is able to react with an aldehyde moiety, in particular R¹⁵ is selected from cysteinyl, threoninyl, 2-mercaptoethanol, cysteamine, ethandithiole, hydroxylamine, O-methylhydroxylamine, N-methylhydroxylamine, dithiothreitol, hydrazine, in particular cysteinyl and N-methylhydroxylamine, more particularly cysteinyl, wherein
amine and/or thiol moieties of the blocking agent may be protected by an independently selected acid labile amine protecting group B, particularly Boc, and/or an acid labile thiol protecting group, particularly trityl.

As described above, the spacer T is generally non-reactive under commonly applied purification conditions with the exception of the removal of protecting groups under acidic conditions. The spacer T may enhance the solubility of the linker molecule. In particular branched spacers that comprise a protected or unprotected amine moiety at R⁹ contribute to an enhanced solubility. Under acidic conditions, the amine protection group B is removed and the amine is protonated.

In certain embodiments, T is a linear or branched spacer comprising at least one of the moieties -C₁₋₁₂alkyl-, -C(=O)-, -C(=O)-NR⁹-, -NR⁹-C(=O)-, -NR⁹-, wherein R⁹ is independently selected from H, C₁₋₄-alkyl, -C₁₋₆-alkyl-NH₂, -C₁₋₆-alkyl-NHB, -C₁₋₆-alkyl-NB₂, in particular from H and C₁₋₂-alkyl, more particularly R⁹ is H, wherein B is an independently selected acid labile amine protecting group.

The spacer T may also comprise a blocking function. In particular a branched spacer may comprise a blocking agent that is suitable to bind to aldehyde moieties. When the solid phase used for peptide purification comprises aldehyde moieties, e.g. agarose beads, the moiety X of the linker compound may be covalently bound to the solid phase, e.g by formation of a oxime bond. Non-reacted aldehyde moieties may cause unwanted side reactions during subsequent purification. To prevent such side reactions, the non-reacted aldehyde moieties of the solid phase can be blocked by the blocking function of the spacer, e.g. a cysteinyl moiety.

In certain embodiments, T is a linear or branched spacer comprising at least one of the moieties -C₁₋₁₂alkyl-, -C(=O)-, -C(=O)-NR⁹-, -NR⁹-C(=O)-, -NR⁹-, wherein R⁹ is independently selected from H, C₁₋₄-alkyl, -R¹⁵, -C₁₋₆-alkyl-R¹⁵, -C₁₋₆-alkyl-NH-R¹⁵, in particular from H and C₁₋₂-alkyl, more particularly R⁹ is H, wherein B is an independently selected acid labile amine protecting group,

R¹⁵ is a blocking agent that is able to react with an aldehyde moiety, in particular R¹⁵ is selected from cysteinyl, threoninyl, 2-mercaptoethanol, cysteamine, ethandithiole, hydroxylamine, O-methylhydroxylamine, N-methylhydroxylamine, dithiothreitol, hydrazine, in particular cysteinyl and N-methylhydroxylamine, more particularly cysteinyl, wherein

amine and/or thiol moieties of the blocking agent may be protected by an independently selected acid labile amine protecting group B, particularly Boc, and/or an acid labile thiol protecting group, particularly trityl.

In certain embodiments, T is selected from -C₁-C₁₂-alkyl-,in particular C₁₋₆-alkyl, more particularly C₁₋₃-alkyl, -R⁵-C(=O)-NR⁹-R⁶-, -C(=O)-NR⁹-R⁶-, -R⁵-NR⁹-C(=O)-R⁶-, -R⁵-C(=O)-O-R⁶-, -C(=O)-O-R⁶-, -R⁵-NR⁹-R⁵-NR⁹C(=O)-R⁶-, particularly from -C₁-C₁₂-alkyl-,in particular C₁₋₆-alkyl, more particularly C₁₋₃-alkyl, -R⁵-NR⁹-C(=O)-R⁶-, -R⁵-C(=O)-NR⁹-R⁶-, -R⁵-NR⁹-R⁵-NR⁹C(=O)-R⁶-, most particularly -R⁵-NR⁹-C(=O)-R⁶-, with R⁵, R⁶ and R⁹ being as defined above.

As described above, the moieties U, V, W and E contribute to the stability of the linker under acidic conditions. For example, linkers comprising a moiety U or V that comprises an amine or a heterocycle such as pyridine are stable under acidic conditions, particularly TFA > 50 %, in the presence of water pH < 0, as the amine or heterocycle is protonated. Furthermore, the protonated linker improves the solubility of the linker-peptide complex. If the pH is higher than the pKa of the linker, the linker will rapidly decompose during the desired release of the peptide as the last step.

V is an electron-withdrawing moiety selected from -NR¹¹-(=O)-, -C(=O)-NR¹¹-, -S(=O)-,-NR¹²-, -piperazinyl-, -pyridinyl, pyrimidinyl, pyrazinyl, pyridazinyl, 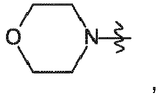 -C(=O)-, - C(=O)-O-, wherein R¹¹ is selected from H and C₁₋₄-alkyl, in particular from H and C₁₋₂-alkyl, more particularly R¹¹ is H, R¹² is selected from H and C₁₋₄-alkyl, in particular from H and C₁₋₂-alkyl, more particularly R¹² is methyl.

V is an electron-withdrawing moiety selected from -NR¹¹-(=O)-, -C(=O)-NR¹¹-, -S(=O)-,-NR¹²-, -piperazinyl-, -pyridinyl, pyrimidinyl, pyrazinyl, pyridazinyl, -C(=O)-, -C(=O)-O-, wherein R¹¹ is selected from H and C₁₋₄-alkyl, in particular from H and C₁₋₂-alkyl, more particularly R¹¹ is H, R¹² is selected from H and C₁₋₄-alkyl, in particular from H and C₁₋₂-alkyl, more particularly R¹² is methyl.

V is an electron-withdrawing moiety selected from -NR¹¹-(=O)-, -C(=O)-NR¹¹-, -S(=O)-,-NR¹²-, -piperazinyl-, -pyridinyl-, pyrimidinyl, wherein R¹¹ is selected from H and C₁₋₄-alkyl, in particular from H and C₁₋₂-alkyl, more particularly R¹¹ is H, R¹² is selected from H and C₁₋₄-alkyl, in particular from H and C₁₋₂-alkyl, more particularly R¹² is methyl.

V is an electron-withdrawing moiety selected from -NR¹¹-(=O)-, -C(=O)-NR¹¹-, -NR¹²-,-piperazinyl-, -pyridinyl-, pyrimidinyl, wherein R¹¹ is selected from H and C₁₋₄-alkyl, in particular from H and C₁₋₂-alkyl, more particularly R¹¹ is H, R¹² is selected from H and C₁₋₄-alkyl, in particular from H and C₁₋₂-alkyl, more particularly R¹² is methyl.

V is an electron-withdrawing moiety selected from -NH-C(=O)-, -C(=O)-HN-, -N-(CH₃)-, - piperazinyl-, -pyridinyl-, pyrimidinyl, pyrazinyl, pyridazinyl.

V is an electron-withdrawing moiety selected from -NH-C(=O)-, -C(=O)-HN-, -N-(CH₃)-,-piperazinyl-, -pyridinyl-, pyrimidinyl.

In certain embodiments, V is an electron-withdrawing moiety selected from -NR¹¹-(=O)-,-C(=O)-NR¹¹-, -S(=O)-, -NR¹²-, -pyridinyl, pyrimidinyl, pyrazinyl, pyridazyl, -C(=O)-, -C(=O)-O-.

In certain embodiments, V is selected from -NR¹¹-(=O)-, -C(=O)-NR¹¹-, -S(=O)-, -NR¹²- and -pyridinyl-, pyrimidinyl, wherein R¹¹ is selected from H and C₁₋₄-alkyl, in particular from H and C₁₋₂-alkyl, more particularly R¹¹ is H, and R¹² is selected from H and C₁₋₄-alkyl, in particular from H and C₁₋₂-alkyl, more particularly R¹² is methyl.

In certain embodiments, V is selected from -NR¹¹-(=O)-, S(=O)-, -NR¹²-, -pyridinyl-, pyrimidinyl, wherein R¹¹ is selected from H and C₁₋₄-alkyl, and R¹² is selected from H and C₁₋₄-alkyl.

In certain embodiments, V is selected from -NR¹¹-(=O)-, S(=O)-, -NR¹²-, -pyridinyl-, pyrimidinyl, wherein R¹¹ is selected from H and C₁₋₂-alkyl, and R¹² is selected from H and C₁₋₂-alkyl.

In certain embodiments, V is selected from -NH-C(=O)-, -N-(CH₃)-, -pyridinyl-, pyrimidinyl.

In certain embodiments, W is selected from -N₃, -S(=O)-R⁸, -S-S-R⁸, -O-CH₂-N₃, -N=N-R⁸, in particular -N₃, -N=N-R⁸, -O-CH₂-N₃, -S-S-R⁸, wherein R⁸ is pyridyl, pyrimidinyl, pyrazinyl, pyridazyl, -C₁-C₆-alkyl or -(CH₂)ₚ-NMe₂, in particular pyridyl or -C₁-C₆-alkyl, with p being 1, 2, 3 or 4.

In case R⁸ is a C₁₋₆-alkyl, the alkyl moiety may be linear or branched, e.g. *tert*-butyl. In particular in case of W being -S-S-R⁸, R⁸ may be pyridyl or -C₁-C₄-alkyl, particularly pyridyl or *tert*-butyl.

Linkers that comprise a moiety W = -S-S-R⁸ may be cleaved with thiols. Therefore, the cleavage of the peptide-linker complex from the synthesis resin used during SPPS under acidic conditions (e.g. TFA > 50 %, in the presence of water pH < 0) may be performed without thiols when such linkers are used for peptide purification.

In certain embodiments, W is selected from -N₃, -S(=O)-R⁸, -S-S-R⁸, -O-CH₂-N₃, -N=N-R⁸, in particular -N₃, -N=N-R⁸, -O-CH₂-N₃, -S-S-R⁸, wherein R⁸ is pyridyl, pyrimidinyl, pyrazinyl, pyridazyl or -C₁-C₆-alkyl or, in particular pyrimidinyl, pyridyl or -C₁-C₆-alkyl, more particularly pyridyl or -C₁-C₆-alkyl.

In certain embodiments, U is selected from phenyl or a five- or six-membered heterocycle, wherein the five- or six-membered heterocycle comprises 1 or 2 heteroatoms.

In certain embodiments, U is selected from phenyl or a five- or six-membered heterocycle, wherein the five- or six-membered heterocycle comprises 1 heteroatom.

In certain embodiments, U is selected from phenyl or a six-membered heterocycle.

In certain embodiments, U is selected from phenyl or a six-membered heterocycle, wherein the six-membered heterocycle comprises 1 or 2 heteroatoms.

In certain embodiments, the five- or six-membered heteroaryl moiety of U comprises 1 or 2 heteroatoms, in particular the five-membered heteroaryl moiety of the moiety U is selected from pyrazole, imidazole, and the six-membered heteroaryl moiety of the moiety U is selected from pyridine, pyridazine, pyrimidine, pyrazine, particularly pyridine.

In certain embodiments, U is selected from phenyl or a six-membered heterocycle, wherein the six-membered heterocycle comprises 1 heteroatom. In certain embodiments, the five-membered heterocycle of the moiety U is selected from pyrazole, imidazole, and the six-membered heterocycle of the moiety U is selected from pyridine, pyridazine, pyrimidine, pyrazine.

In certain embodiments, U is selected from phenyl, pyridine, pyridazine, pyrimidine, pyrazine, particularly phenyl or pyridine, more particularly phenyl.

In certain embodiments, U is selected from a moiety of formula 5, 6, 7 or 8, 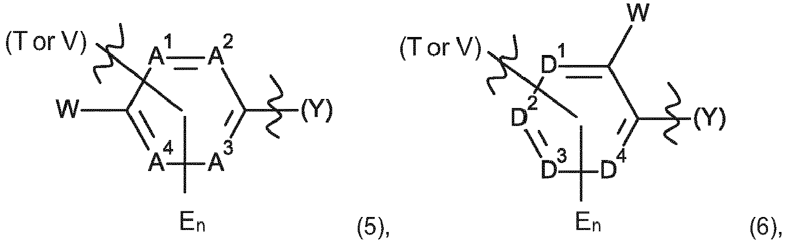 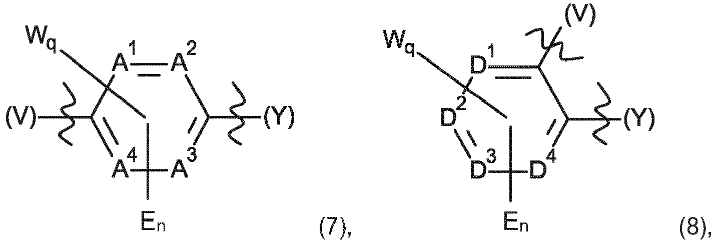 wherein
T, V, Y, W and E are defined as described above,
in case of formula 5 and 6, U is bound to the moiety T or V,
in case of formula 7 and 8, U is bound to the moiety V,
A¹, A², A³, A⁴ and D¹, D², D³, D⁴ are independently from each other selected from C, N, S and O, in particular from C and N, wherein 2 to 4 moieties of A¹, A², A³ and A⁴ or of D¹, D², D³ and D⁴ are C, particularly 3 or 4 moieties of A¹, A², A³ and A⁴ or of D¹, D², D³ and D⁴ are C, more particularly all moieties A¹, A², A³ and A⁴ or of D¹, D², D³ and D⁴ are C,
n is
   in case of formulas 5 and 6 an integer between 0 and 3,
   in case of formulas 7 and 8 an integer between 0 and 4,
q is an integer between 0 and 4, wherein the sum of n and q is equal or lower than 4.

In certain embodiments, U is selected from a moiety of formula 5 or 6.

In certain embodiments, U is selected from a moiety of formula 5, 6, 7 or 8, wherein
T, V, Y, W and E are defined as described above,
in case of formula 5 and 6, U is bound to the moiety T or V,
in case of formula 7 and 8, U is bound to the moiety V,
A¹, A², A³, A⁴ and D¹, D², D³, D⁴ are independently from each other selected from C, N, S and O, in particular from C and N, n is
   in case of formulas 5 and 6 an integer between 0 and 2,
   in case of formulas 7 and 8 an integer between 0 and 2, in particular 0 and 1,
q is an integer between 0 and 2, in particular 0 and 1.

In certain embodiments, U is selected from a moiety of formula 9, 10, 11 or 12, in particular of formula 9 or 10, 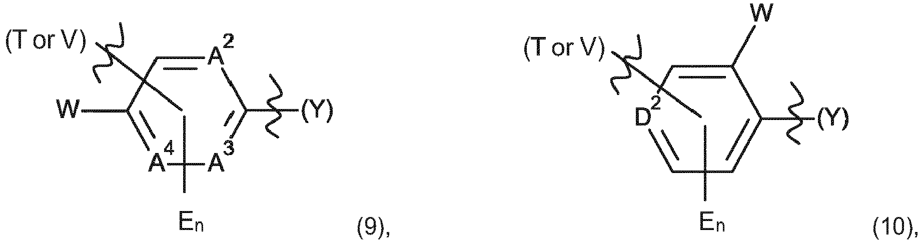 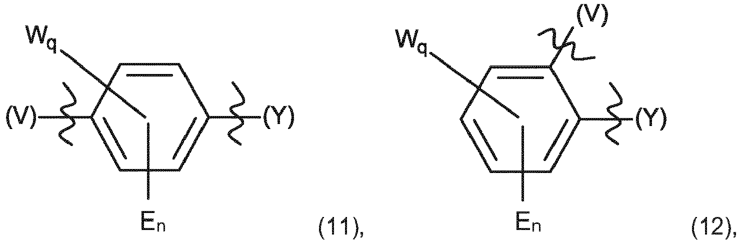 wherein
T, V, Y, W, E, q and n are defined as described above,
in case of formula 9 and 10, U is bound to the moiety T or V,
in case of formula 11 and 12, U is bound to the moiety V,
all moieties A², A³ and A⁴ are C or two of A², A³ and A⁴ are C and the other two of A², A³ and A⁴ is N, in particular A² and A³ are both C, and
D² is C or N, in particular C.

In certain embodiments, U is selected from a moiety of formula 9, 10, 11 or 12, 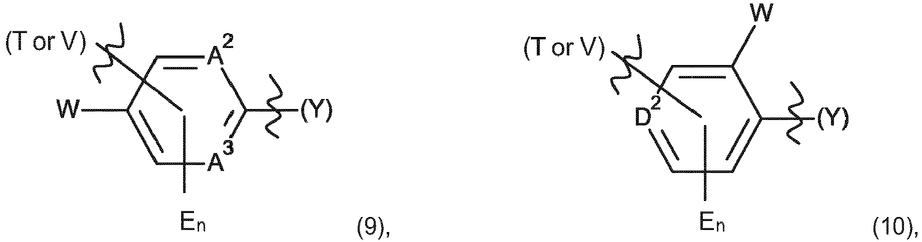 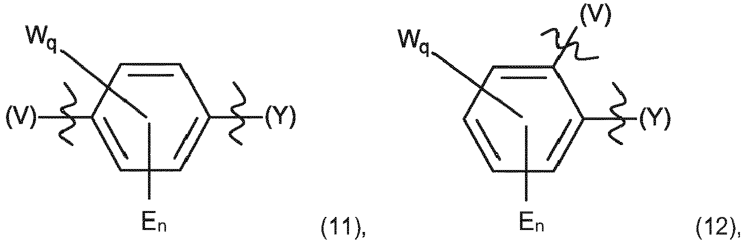 wherein
T, V, Y, W, E, q and n are defined as described above,
in case of formula 9 and 10, U is bound to the moiety T or V,
in case of formula 11 and 12, U is bound to the moiety V,
A² and A³ are both C or one of A² and A³ is C and the other one of A² and A³ is N,
D² is C or N.

If the moiety U comprises a N-containing heteroaryl, the N atom is protonated under acidic conditions and increases therefore the solubility of the linker molecule.

In certain embodiments, U is selected from a moiety of formula 13, 14, 15, 16, 17,18, 19, 20 or 21, in particular of formula 13 to 19, more particular of formula 15 or 19, 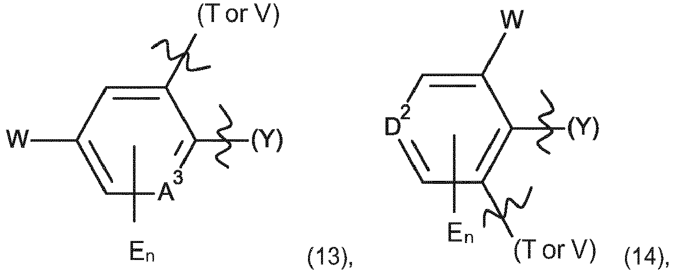 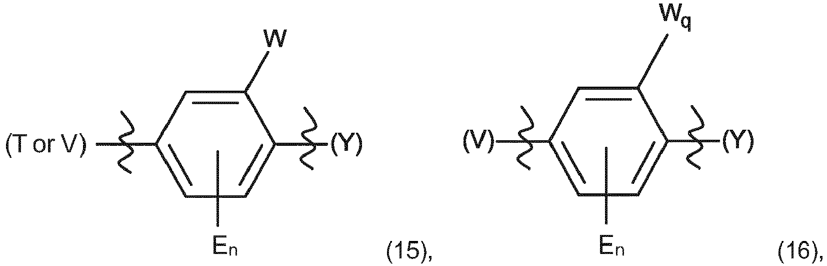 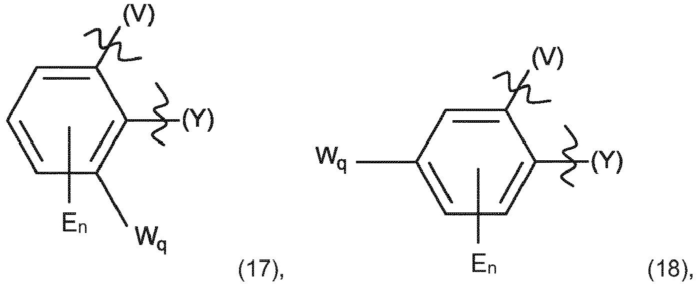 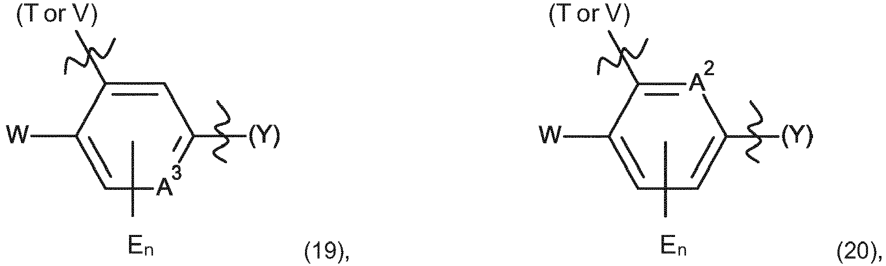 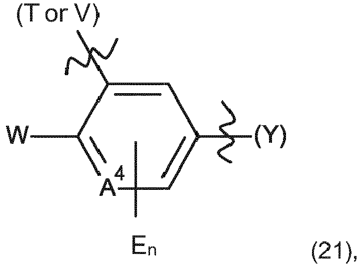 wherein
T, V, Y, W, E, q and n are defined as described above,
in case of formula 13, 14 and 15, U is bound to the moiety T or V,
in case of formula 16, 17 and 18, U is bound to the moiety V,
A², A³ and A⁴ is C or N, in particular C,
D² is C or N.

In certain embodiments, U is selected from a moiety of formula 13, 14, 15, 16, 17 or 18, 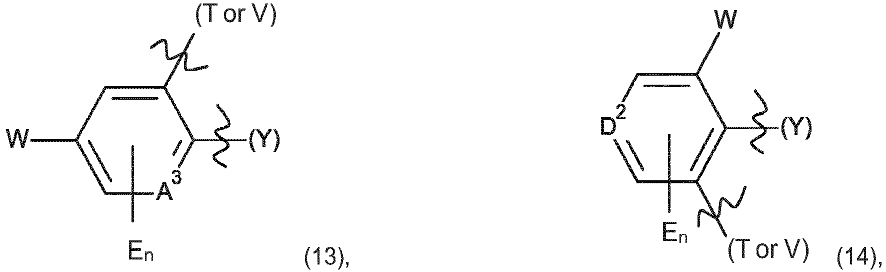 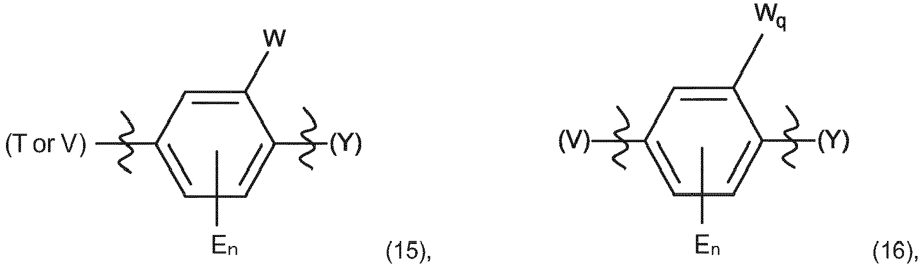 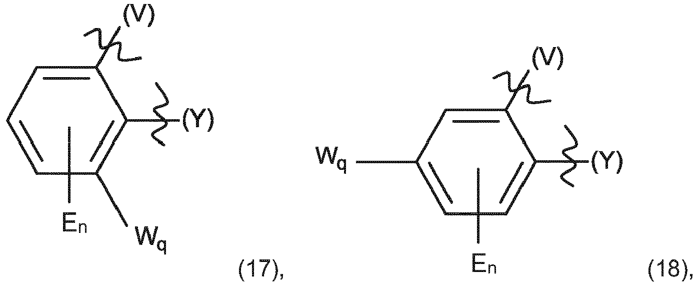 wherein
T, V, Y, W, E, q and n are defined as described above,
in case of formula 13, 14 and 15, U is bound to the moiety T or V,
in case of formula 16, 17 and 18, U is bound to the moiety V,
A³ is C or N,
D² is C or N.

In certain embodiments, Z is selected from: -F, -Cl, -Br, -I, -N₃, -OH, -O(C=O)CH₂(C=O)OH-SR¹⁴, -OCF₃, -OCH₂CF₃, -OSO₂CF₃, -SO₂C₆H₄CH₃, -SO₂CF₃, -SO₂CH₃ 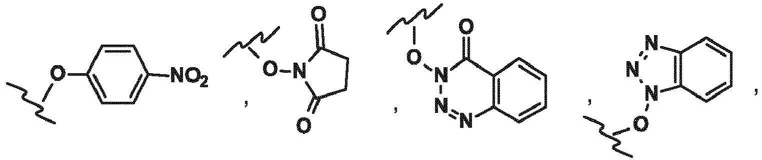 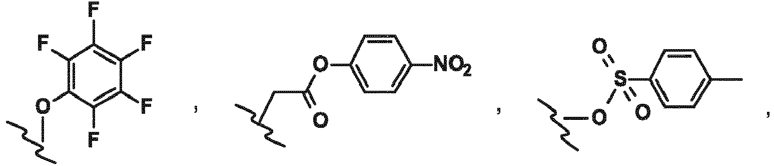 in particular -OH, -CI, 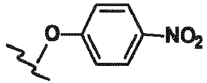 or 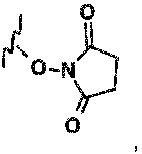 in particular -OH, 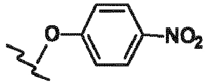 or 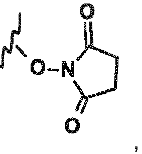 wherein R¹⁴ is an C₁-C₆-alkyl-, an arylic- or benzylic substituent.

In certain embodiments, Z is selected from: -F, -CI, -Br, -I, -N₃, -OH, -SR¹⁴, -OCF₃,-OCH₂CF₃, -OSO₂CF₃, -SO₂C₆H₄CH₃, -SO₂CF₃, -SO₂CH₃ 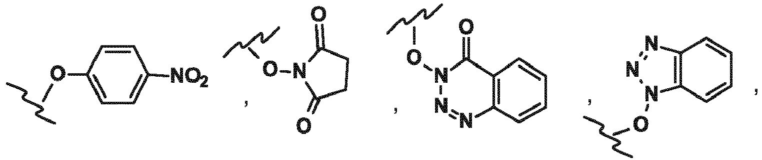 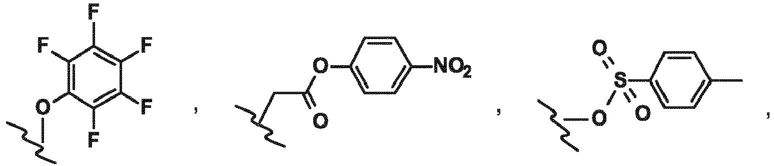 in particular -OH, -CI, 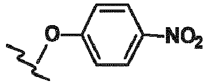 or 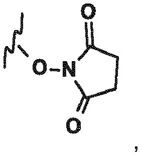 in particular -OH, 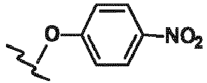 or 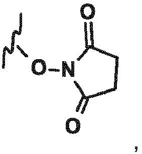 wherein R¹⁴ is an C₁-C₆-alkyl-, an arylic- or benzylic substituent.

In certain embodiments, the compound of formula 1 is selected from a compound of formula X1, X2, X3, X4, X5, X7, X8, X9, X10, X11, X12, X14, X15, X16, X17, X18, X19, X20, X21, X22, X23 X24, X25 or X26.

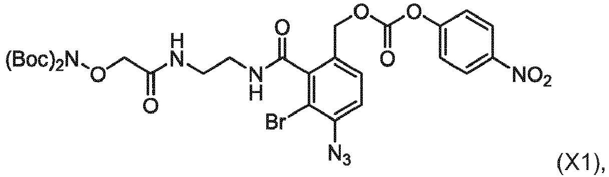

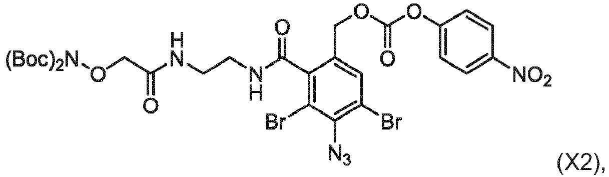

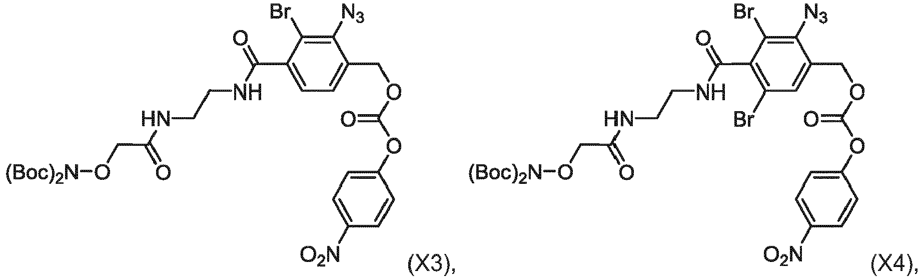

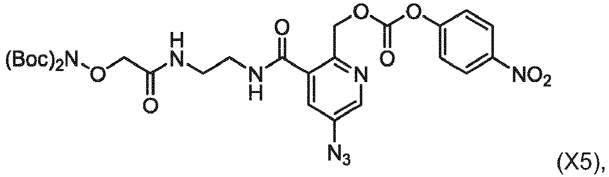

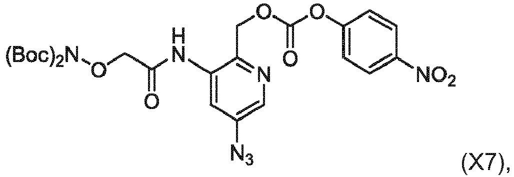

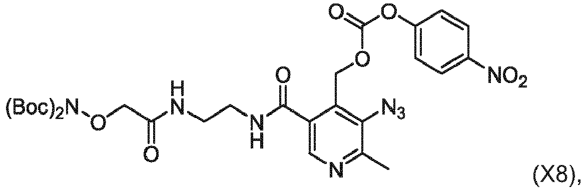

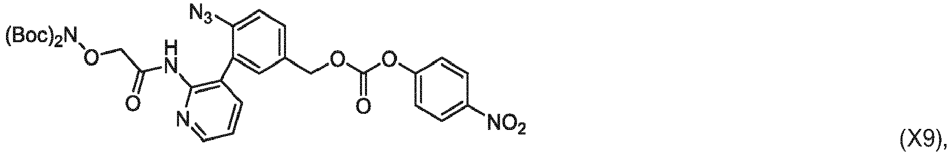

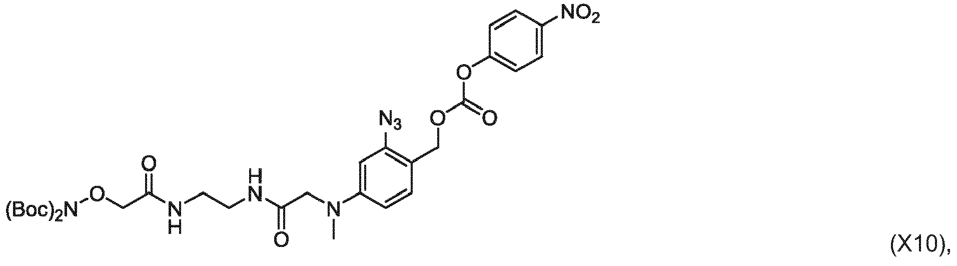

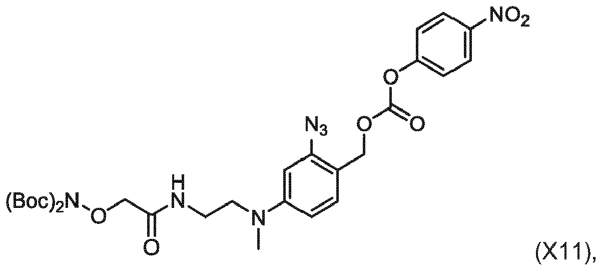

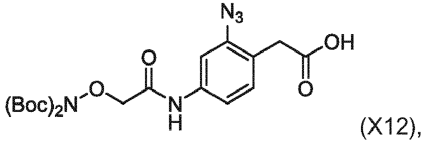

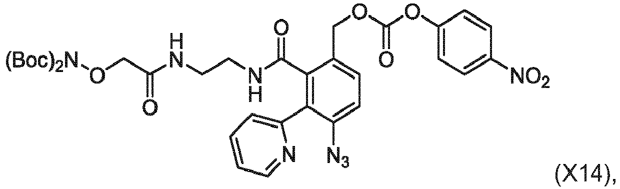

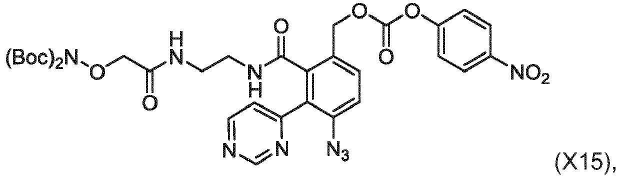

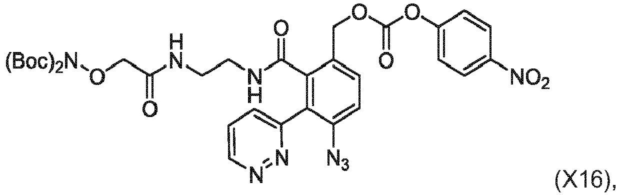

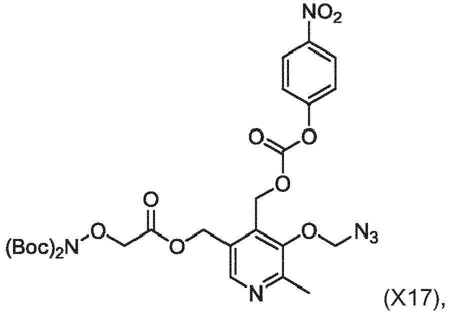

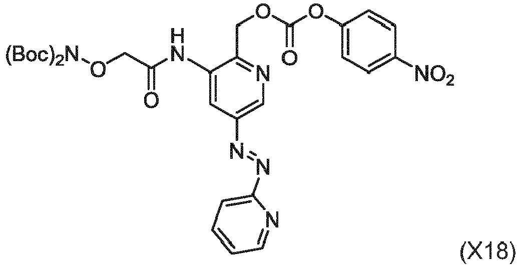

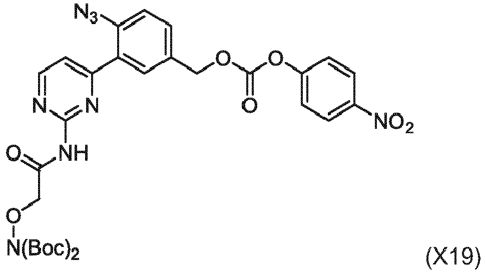

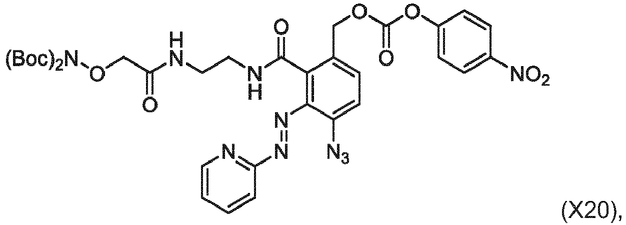

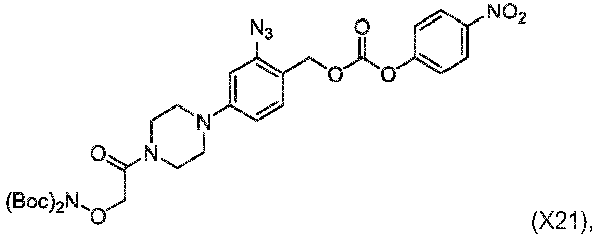

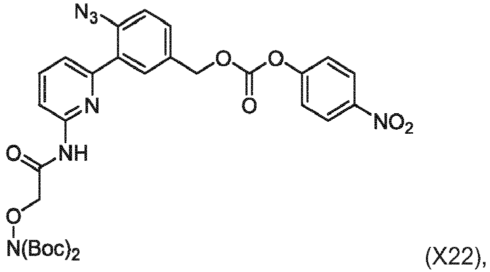

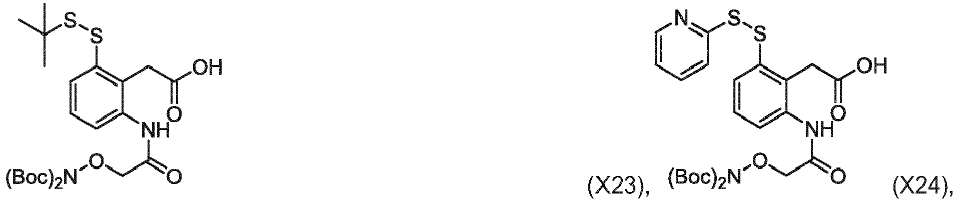

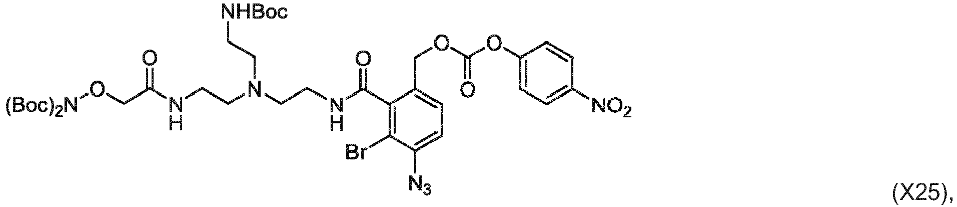

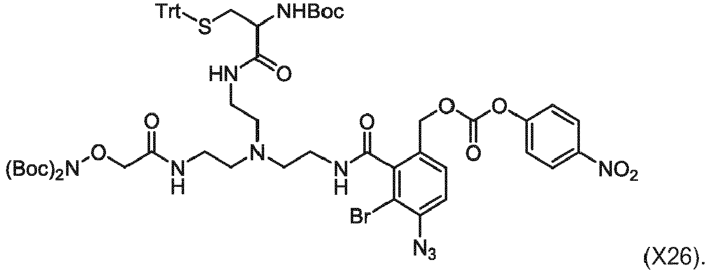

In certain embodiments, the compound of formula 1 is a compound of formula X9, X12, X19, X22, X8, X18, X1, X14, X15, X20, X7, X11, X17, X21, X2, X16, X23 or X24.

In certain embodiments, the compound of formula 1 is a compound of formula X9, X12, X19, X22, X8, X18, X1, X14, X15 or X20.

In certain embodiments, the compound of formula 1 is a compound of formula X9, X12, X19, X22 or X8.

A second aspect of the invention aims at a method for purifying peptides.

According to a second aspect of the invention, a method for purifying peptides is provided. The method comprises the steps of
- providing a crude linker-modified peptide, wherein the crude peptide is covalently bound to a linker molecule according to the first aspect of the invention,
- in a coupling step, coupling the linker-modified peptide with a solid support yielding an immobilized linker-modified peptide,
- in a releasing step, releasing the peptide by adding a reducing agent under acidic conditions.

The linker molecule according to the first aspect of the invention may be used in a method for purifying peptide. In a first step, a crude peptide mixture is contacted with a linker molecule according to the first aspect of the invention and a crude peptide is coupled to a linker molecule yielding a crude linker-modified peptide. The crude peptide is coupled to a linker molecule by standard methods that are generally known to a general expert in the field of chemistry, biochemistry or pharmacy.

During purification, the stability of the linker molecule can be addressed by adjusting the pH. Under acidic conditions (in particular at a pH below the pKa of the most basic heteroatom of the linker molecule), the linker molecule is stable due to the fact that this heteroatom is protonated and withdraws electrons. Upon binding of the linker to a peptide and immobilization on a solid support, the peptide is released via a reduced intermediate under reducing conditions, e.g. adding a reducing agent such as triphenylphoshine under acidic conditions. The reduced intermediate is characterized by a reduced linker moiety, e.g. an azaylide. The linker moiety of the reduced intermediate decays over time or upon a trigger, such as increasing the pH, in particular to pH > pKa of the most basic heteroatom of the linker moiety of the reduced intermediate. Thus, all steps before the release of the peptide (coupling, optional washing and reduction of the linker) are performed under acidic conditions. Upon increase of the pH to a pH above the pKa of the most basic heteroatom of the linker moiety of the reduced intermediate, the reduced intermediate linker moiety decays by 1.4/1.6 elimination reaction or nucleophilic attack and the peptide is released.

The most basic heteroatom of the linker molecule/linker moiety of the reduced intermediate relates to the following: For example, the moiety U consists of a pyridine substituted by -N₃. The heteroatom N of pyridine has a pKₐ ∼ 5 which is higher than the pKₐ of the -N₃ moiety or the reduced -N₃ moiety (pKₐ approx. 4.6). Thus, the most basic heteroatom is the N of the pyridine moiety. By shifting the pH to pH > 5, the linker moiety of the reduced intermediate undergoes an elimination reaction and the peptide is released.

In certain embodiments, the pH in all steps before adding a reducing agent is pH < pKa of the most basic heteroatom of the linker moiety.

In certain embodiments, the peptide is released via an intermediate.

In certain embodiments, the peptide is released via a reduced intermediate characterized by a reduced linker moiety of the immobilized linker-modified peptide by adding a reducing agent under acidic conditions.

In certain embodiments, a reduced intermediate characterized by a reduced linker moiety of the immobilized linker-modified peptide is achieved in the releasing step and the peptide is released from said reduced intermediate by a trigger, in particular a change in temperature and/or pH, more particularly by increasing the pH to pH > pKa of the most basic heteroatom of the linker moiety of the reduced intermediate

The peptide is released from the intermediate spontaneously or by a trigger.

In certain embodiments, the trigger is a change in temperature and/or pH.

In certain embodiments, the change in temperature is an increase of the temperature from ambient temperature (20 °C to 30 °C) to a higher temperature, wherein the higher temperature does not exceed 100 °C, particularly 70 °C, more particularly 50 °C.

In certain embodiments, the trigger is a shift in pH, in particular increasing the pH.

In certain embodiments, the peptide is released from a reduced intermediate by increasing the pH to pH > pKa of the most basic heteroatom of the linker moiety of the reduced intermediate.

If the reduced intermediate is stable under acidic conditions, an additional washing step can be performed to remove excess reducing agent. As a reducing agent may react not only with the linker moiety but also with the peptide, unwanted side reactions between the reducing agent and the peptide are reduced by the additional washing step. Furthermore, none volatile reducing agents or products of their usage are also impurities, that would have to be removed by an additional purification step.

In certain embodiments, the reducing agent is removed by washing after the reduced intermediate is formed and before the peptide is released from the reduced intermediate.

In certain embodiments, the reducing agent is removed using MeCN.

In certain embodiments, the linker molecule according to the first aspect of the invention comprises a moiety W and/or E that comprises an azide (-N₃) moiety.

In certain embodiments, the linker-modified peptide is additionally bound to a synthesis resin and the synthesis resin is cleaved off before step (b) is performed, in particular at pH < pKa of the most basic heteroatom of the linker molecule.

The cleavage of the peptide off the synthetic support is achieved by usage of TFA, finalized by precipitating the peptide (e.g. after 2-8h) out of the TFA mixture providing a crude peptide mixture. For the precipitation, cold ether (Et₂O, iPr₂O, MeOtBu, THF/Hexane (1:1)) can be used.

The crude peptide mixture is dissolved in a suitable organic solvent, particular DMSO, and a buffer system, particularly 10 volume percent of sodium citrate buffer 0.1 M at pH 4.5, is added.

The solid support for the coupling step is an aldehyde modified solid support, in particular agarose beads, polylysine, polyethylene glycol, polyamide, polystyrene and copolymers of those, to which the dissolved crude peptide mixture is added.

In certain embodiments, the solid support comprises aldehyde moieties.

In certain embodiments, non-reacted aldehyde moieties of the solid support are blocked using a blocking agent after performing step (b).

In certain embodiments, the blocking agent reacts with aldehyde moieties of the solid support and comprises a thiol and/or amine moiety.

In certain embodiments, the blocking agent is selected from cysteine, threonine, 2-mercaptoethanol, cysteamine, ethandithiole, hydroxylamine, O-methylhydroxylamine, N-methylhydroxylamine, dithiothreitol, hydrazine.

In certain embodiments, the blocking agent is selected from cysteine and N-methylhydroxylamine.

The coupled product is washed, particularly with DMSO, 6 M guanidium hydrochloride, EtOH/water (7:3) with 0.1 M NaCl, water, MeCN.

In certain embodiments, step (c) is performed at pH < pKa of the most basic heteroatom of the linker molecule.

In certain embodiments, the reducing agent is selected from triphenylphosphine, trimethylphosphine, triethylphosphine, tributylphosphine or tris(2-carboxyethyl)phosphine, trimethyl phosphite, triethyl phosphite, tributyl phosphine, diethyl phosphite, 5,5'-Dithiobis(2-nitrobenzoic acid), sodium dithionite (Na₂S₂O₄), ethandithiole, Propandithiol, dithiothreitol, Na₂S, NaSH, glutathione, 2,2'-dithiodipyridine, BH₃, 4,4,5,5-tetramethyl-1,3,2-dioxaborolane, catechol borane, borane tetrahydrofuran, borane dimethyl sulfide, borane dimethylamine complex, borane triphenylphosphine complex, borane tert-butylamine, LiAlH₄, LiBH₄, NaBH₄, NaBH₃CN, NaBH(OMe)₃, NaBH(OCCH₃)₃. LiAlH(OCMe₃)₃, hydroquinone, sodium ascorbate, ascorbic acid, hydrazine, NH=NH, formaldehyde.

In certain embodiments, the reducing agent is selected from triphenylphosphine, tributylphosphine, trimethylphsophine, triethylphosphine or tris(2-carboxyethyl)phosphine, sodium dithionite (Na₂S₂O₄), borane dimethyl sulfide, borane triphenylphosphine complex, NaBH₄, ascorbic acid.

In certain embodiments, the reducing agent is selected from triphenylphosphine, trimethylphsophine, triethylphosphine or tris(2-carboxyethyl)phosphine, sodium dithionite (Na₂S₂O₄), borane dimethyl sulfide, borane triphenylphosphine complex, NaBH₄, ascorbic acid.

In certain embodiments, the reducing agent is selected from triphenylphosphine, sodium dithionite (Na₂S₂O₄), borane dimethyl sulfide, borane triphenylphosphine complex, NaBH₄, ascorbic acid.

In certain embodiments, the reducing agent is selected from triphenylphosphine and trimethylphosphine.

In certain embodiments, the reducing agent is triphenylphosphine.

In certain embodiments, method comprises the steps of
- providing a crude linker-modified peptide, wherein the crude peptide is covalently bound to a linker molecule according to the first aspect of the invention, wherein the linker molecule comprises a moiety W and/or E that comprises an azide moiety,
- in a coupling step, coupling the linker-modified peptide with a solid support yielding an immobilized linker-modified peptide,
- in a releasing step, releasing the peptide by adding a reducing agent under acidic conditions.

In certain embodiments, method comprises the steps of
- providing a crude linker-modified peptide, wherein the crude peptide is covalently bound to a linker molecule according to the first aspect of the invention, wherein the linker molecule comprises a moiety W and/or E that comprises an azide moiety,
- in a coupling step, coupling the linker-modified peptide with a solid support yielding an immobilized linker-modified peptide,
- in a releasing step, releasing the peptide by adding a reducing agent under acidic conditions yielding a reduced intermediate followed by increasing the pH pH > pKa of the most basic heteroatom of the linker moiety of the reduced intermediate.

In certain embodiments, method comprises the steps of
- providing a crude linker-modified peptide, wherein the crude peptide is covalently bound to a linker molecule according to the first aspect of the invention, wherein the linker molecule comprises a moiety W and/or E that comprises an azide moiety,
- in a coupling step, coupling the linker-modified peptide with a solid support yielding an immobilized linker-modified peptide,
- in a releasing step, releasing the peptide by adding a triphenylphosphine, trimethylphosphine, triethylphosphine or tris(2-carboxyethyl)phosphine, particularly triphenylphosphine, under acidic conditions yielding a reduced intermediate followed by increasing the pH pH > pKa of the most basic heteroatom of the linker moiety of the reduced intermediate.

According to a sub-aspect of the second aspect of the invention, a method for purifying a crude peptide prepared by solid-phase peptide synthesis is provided.

In certain embodiments, the method for purifying peptides comprises the steps of
a) providing a peptide bound to a synthetic resin, wherein the peptide is additionally covalently bound to a linker molecule according to claim 1, wherein the linker molecule comprises a moiety W and/or E that comprises an azide moiety,
b) cleaving of the peptide off the synthetic resin
c) coupling the cleaved peptide mixture with a solid support
d) releasing the peptide with triphenylphosphine, or trimethylphosphine, triethylphosphine or tris(2-carboxyethyl)phosphine, particularly triphenylphosphine.

The cleavage of the peptide off the synthetic support is achieved by usage of TFA, finalized by precipitating the peptide (e.g. after 2-8h) out of the TFA mixture providing a crude peptide mixture. For the precipitation, cold ether (Et₂O, iPr₂O, MeOtBu, THF/Hexane (1:1)) can be used.

The crude peptide mixture is dissolved in a suitable organic solvent, particular DMSO, and a buffer system, particularly 10 volume percent of sodium citrate buffer 0.1 M at pH 4.5, is added.

The solid support for the coupling step is an aldehyde modified solid support, in particular agarose beads, polylysine, polyethylene glycol, polyamide, polystyrene and copolymers of those, to which the dissolved crude peptide mixture is added.

The coupled product is washed, particularly with DMSO, 6 M guanidium hydrochloride, EtOH/water (7:3) with 0.1 M NaCl, water, MeCN.

In certain embodiments, triphenylphosphine, trimethylphosphine, triethylphosphine or tris(2-carboxyethyl)phosphine, particularly triphenylphosphine, is added in MeCN/AcOH (9:1). In certain embodiments, the addition is made for 15 min.

In certain embodiments, triphenylphosphine, trimethylphosphine, triethylphosphine or tris(2-carboxyethyl)phosphine is added in MeCN/AcOH/H₂O (90:5:5) and/or wherein after the addition of triphenylphosphine, trimethylphosphine, triethylphosphine or tris(2-carboxyethyl)phosphine, particularly triphenylphosphine, the formed azaylide is washed out, in particular with MeCN or MeCN/H₂O (9:1).

In certain embodiments, triphenylphosphine, trimethylphosphine, triethylphosphine or tris(2-carboxyethyl)phosphine, particularly triphenylphosphine, is added in MeCN/AcOH/H₂O (90:5:5). In certain embodiments, the addition is made for 15 min.

In certain embodiments, after the addition of triphenylphosphine, trimethylphosphine, triethylphosphine or tris(2-carboxyethyl)phosphine the formed azaylide is washed, in particular with MeCN or MeCN/H₂O (9:1).

In certain embodiments, after the addition of triphenylphosphine, trimethylphosphine, triethylphosphine or tris(2-carboxyethyl)phosphine the formed azaylide is washed, in particular with MeCN.

In certain embodiments, the formed azaylide is hydrolyzed, in particular with H₂O/TFA. The ration can be 99.95% water to 50% water.

When the linker, in particular U, E, W or V are nitrogen containing heterocycles the pH should be above the pKa of the heterocyclic moiety, this might be already the case in the TFA-water hydrolysis mixture or by addition of a buffer solution with the desired pH.

In certain embodiments, an elution step is performed after the hydrolysis, in particular with TFA/H₂O, more particular with a ratio of 9:1.

In certain embodiments, an elution step is performed after the hydrolysis, in particular with TFA/H₂O, more particular with a ratio of 95:5.

In certain embodiments, the hydrolysis product is precipitated, in particular by adding cold ether, more particular Et₂O, iPr₂O, MeOtBu, THF/Hexane (1:1).

### Terms and definitions

In the context of the present invention, "electron-withdrawing group" or "EWG" is any chemical group that is able to draw electrons away from its connected atom or arylic system through inductive or mesomeric effect.

In the context of the present invention, Hammett constants are constants as calculated and described in Hansch and Taft (1991), Chem. Rev. 91:165-195. A positive Hammett constant reflects the ability of a substituent to exert an electron withdrawing effect on a phenyl moiety and a negative value indicates that a substituent exerts and electron donating effect. The electron withdrawing effect is stronger the larger the Hammett constant is. Hammett constants are empirically determined constants for substituents of phenyl moieties in meta (σₘ) and para position (σₚ). In the context of the present invention, the position is determined in relation to the binding of the moiety Y. For substituents in ortho position, Hammett values for the para position are a good approximation and therefore used in the context of the present invention to calculate the sum of Hammett values of the substituents V, W and E.

In the context of the present invention, the term "under acidic conditions" relates to a pH below pH 7, in particular a pH below the pKa of the linker, more particularly TFA > 50 %, in the presence of water pH < 0.

In the context of the present invention, the term alkyl refers to a linear or branched saturated hydrocarbon. For example, the term C₁₋₁₂alkyl signifies saturated linear or branched hydrocarbon having 1, 2, 3, 4, 5, 6, 7, 8, 9, 10, 11 or 12 carbon atoms. Non-limiting examples for a C₁-C₄ alkyl are methyl, ethyl, propyl, n-butyl, 2-methylpropyl and *tert*-butyl.

### Brief description of the figures

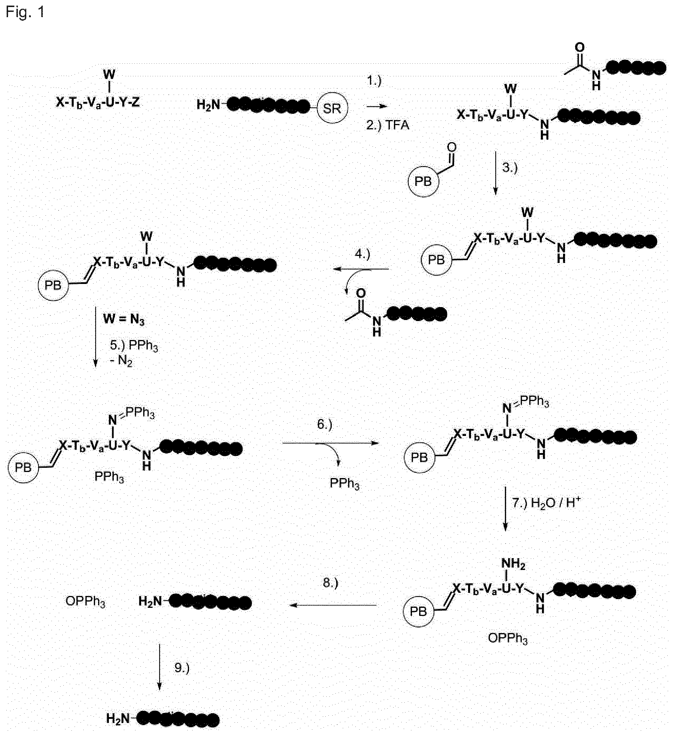

Figure 1 shows a schematic representation of the inventive peptide purification by usage of linkers of the type X-T_{b}-Vₐ₋U(W)-Y-Z, wherein W as N₃ and the method is described in claim 10. SR = synthetic resin, PB = purification beads; 1.) 4 eg. X-T_{b}-Vₐ₋U(W)-Y-Z, 6 eq. Oxyma, 6 eq. DIEA, 2h. 3.) Dissolution of crude peptide with DMSO and addition of 10 volume % of NaCitrate pH 4.5., 90 min. 4.) washing. 5.) PPh3 in MeCN/AcOH 9:1, 15 min. 6.) Washing with MeCN. 7) Hydrolysis with H₂O/TFA. 8.) 1,6- or 1,4-elimination. 9.) Final ether precipitation.

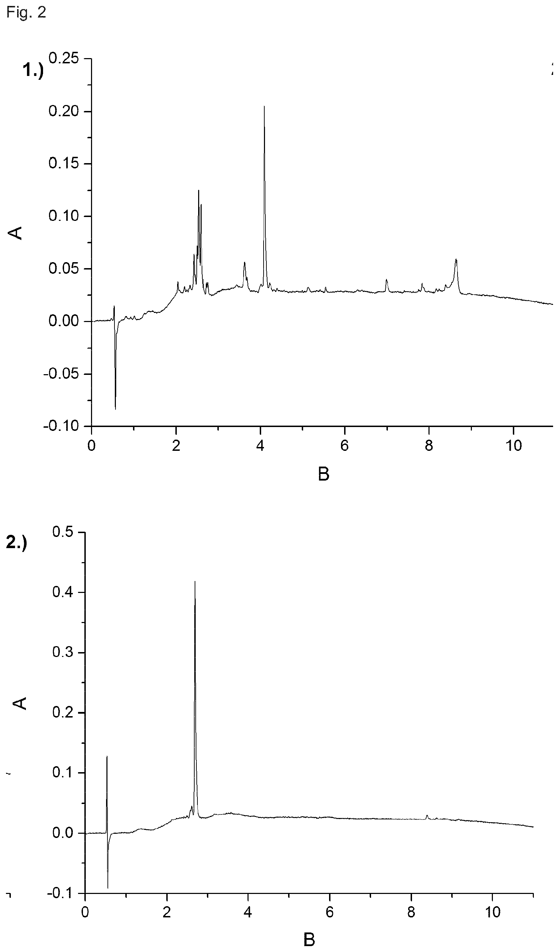

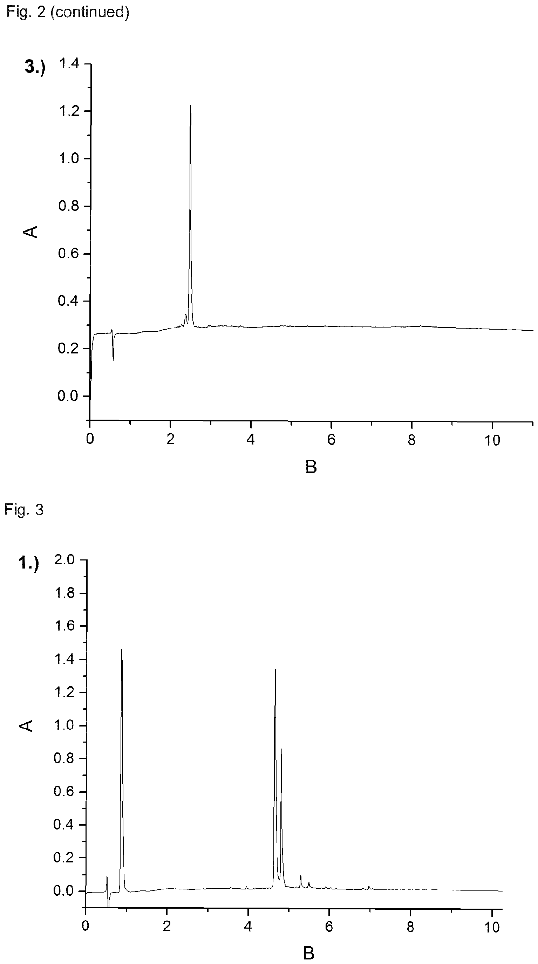

Figure 2 shows an example of the inventive peptide purification of peptide P1 (H-ARTKQTARKSTGGKA-OH) by usage of inventive linker molecule X1 and X2. A = absorption (210 nm), B = time / min; 1.) Chromatogram of crude peptide sample before linker coupling to P1. 2.) Chromatogram of P1 after purification by usage of linker X1 and method as described in claim 10. 3.) Chromatogram of P1 after purification by usage of linker X2 and method as described in claim 10.

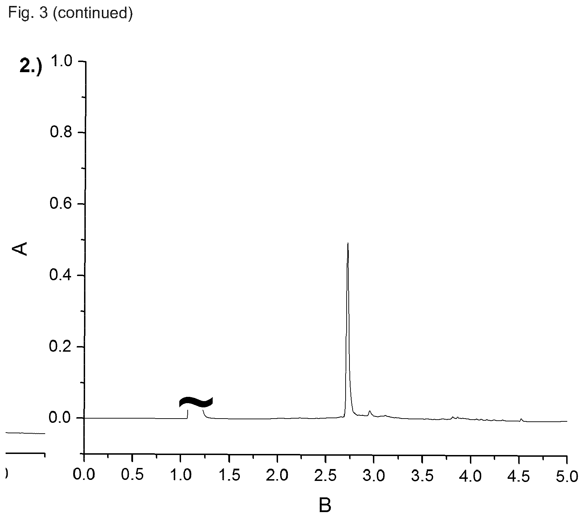

Figure 3 shows an example of the inventive peptide purification of peptide P2 (H-AKADEVSLHKWYG-NH₂) by usage of inventive linker molecule X2. A = absorption (210 nm), B = time / min; 1.) Chromatogram of crude peptide sample before linker coupling to P2. 2.) Chromatogram of P2 after purification by usage of linker X2 and method as described in claim 10. ∼ 3-amino benzoic acid used as internal standard for peptide quantification.

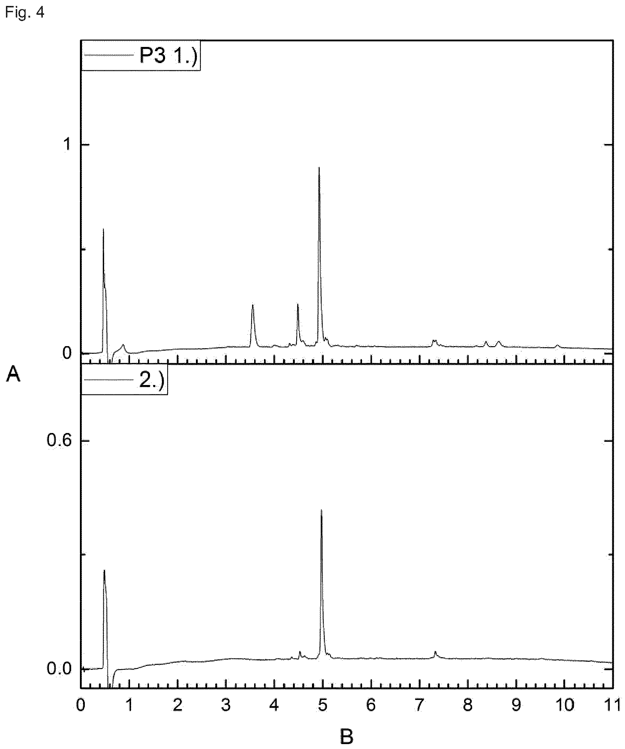

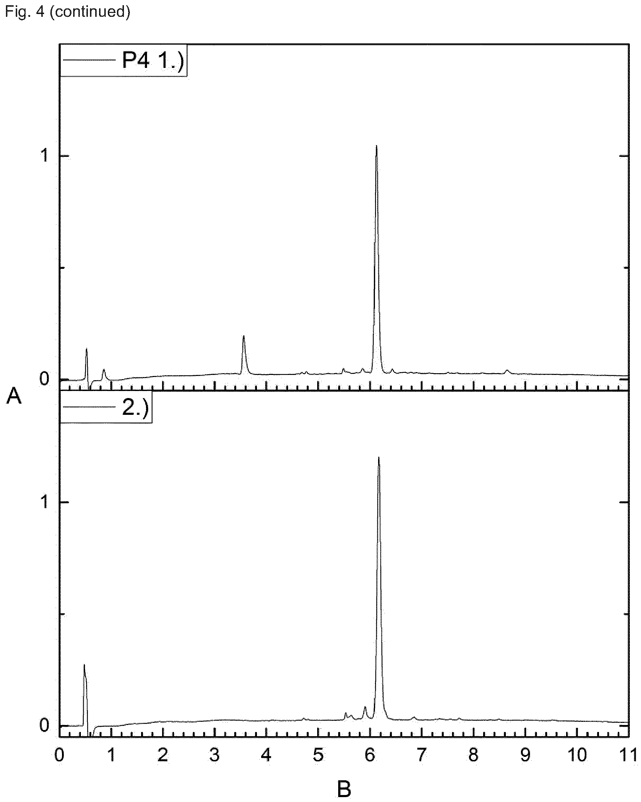

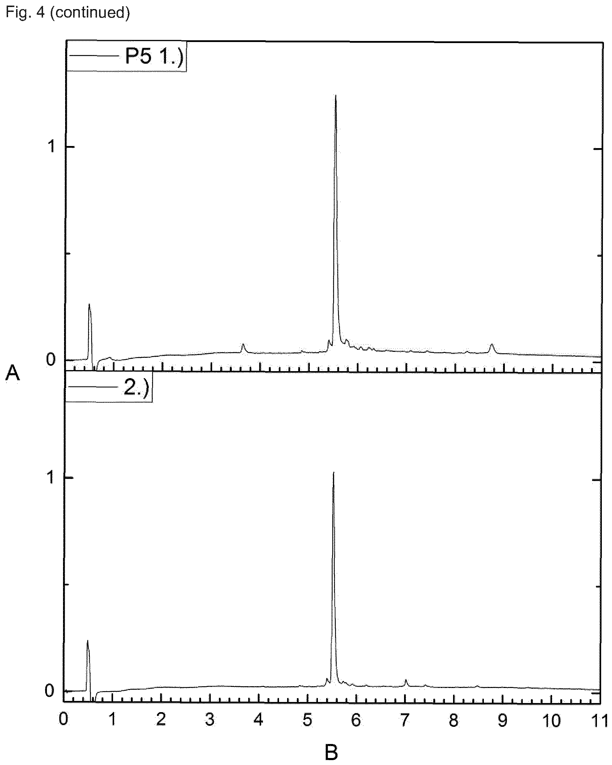

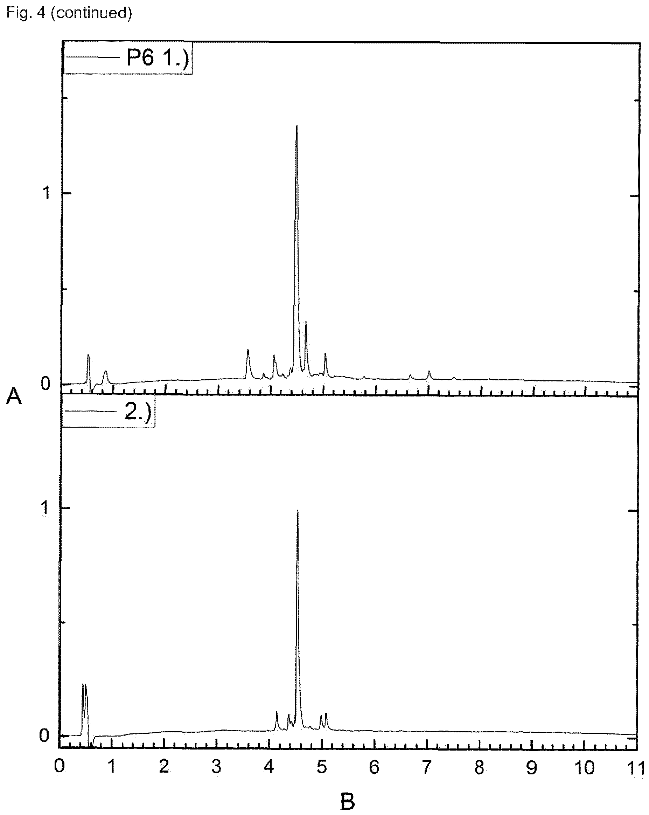

Fig. 4 shows four examples of the inventive peptide purification of peptides P3 (H-YFTGSEVENVSVNVH-NH₂), P4 (H-PSNPFYEALST-NH₂), P5 (H-DAEFRHDSGYEVHHQKLVFF-NH₂) and P6 (H-CKADEVSMHKWYG-NH₂) by usage of inventive linker molecule X1. A = absorption (210 nm), B = time / min; 1.) Chromatogram of crude peptide sample before linker coupling to peptide. 2.) Chromatogram of peptide after purification by usage of linker X1 and method as described in claim 10.

### Examples

### Example 1: purification of naturally occurring and research peptides P1 and P2

The inventive method for the purification of peptides was applied to two peptides of different polarity, these were H-ARTKQTARKSTGGKA-OH (SEQ ID NO: 1) (P1) fragment 2-16 of the Histone H3 protein and H-AKADEVSLHKWYG-NH₂ (SEQ ID NO: 2) (P2) is a peptide sequence intended for research.

The peptide sequences were synthesized under standard solid phase peptide synthesis conditions, whereby the synthetic resin was treated with acetic anhydride and pyridine after each amino-acid coupling to block unreacted amino groups. Linker X1 was coupled to P1 on resin by usage of 4 eq. linker, 6 eq. oxyma and 6 eq. diisopropylamine (DIEA) in DMF for 2 h. The inventive method is shown in Figure 1. Linker X2 was coupled to P1 and P2 on resin by usage of 4 eq. linker, 6 eq. oxyma and 6 eq. DIEA for 2 h. Thereafter, peptides were cleaved of the synthetic resin by a mixture of TFA / PhOH / PhSH / H₂O / ethanedithiol (EDT, 82.5:5:5:2.5) The crude peptide mixture was dissolved in Dimethylsulfoxide (DMSO). Aldehyde modified agarose beads were washed each 3x with water and NaCitrate buffer 0.1 M at pH 4.5. To the DMSO solutions of the peptides 10 vol.% of NaCitrate buffer was added, then the solution was applied to the agarose beads for 90 minutes, what immobilized the desired peptide quantitatively on the agarose beads. Subsequently washing each 3x was performed with 8 M urea, DMSO, EtOH/water (7:3), 0.1M NaCl, water, MeCN to remove any acetylated termination sequences and other impurities. The cleavage of the immobilized linker was carried out by treating the agarose resin with 50 mg PPh₃ per mL MeCN/AcOH (9:1). Afterwards the support was rinsed 4x with MeCN and a solution of H₂O/MeCN/TFA (70:29:1) was added for 180 min. Thereafter, the supernatant was filtered into a centrifuge tube and the support was rinsed 3x with TFA/H₂O (9:1) into the same tube. Et₂O was added 10-fold to initiate precipitation and the peptide was gained by centrifugation of the tube and disposal of the organic supernatant.

UPLC-MS was used to verify the purity of the individual phases. The UPLC-chromatograms of the non-purified (without linker molecule) and purified peptides are shown in Figure 2 and Figure 3. Identities of the peptides were confirmed by ESI-MS. The amount of 26 mg (recovery 62%) of Peptide P1 was gained by usage of X1 in a 93% purity (originally 41%) in the purification of 100 µmol P1. By usage of X2 linker 21 mg (recovery 50%) was gained in 93% purity. P2 was purified in 5 µmol scale by usage of X2 linker, whereby 4 mg (recovery 73%) was gained with a purity of 95% (originally 55%).

**Table 1**

| No. | sequence and linker | UV-purity before | UV-purity after method | recovery |
|---|---|---|---|---|
| P1 | H-ARTKQTARKSTGGKA-OH with X1 | 41% | 93% | 62% |
| P1 | H-ARTKQTARKSTGGKA-OH with X2 | 41% | 93% | 50% |
| P2 | H-AKADEVSLHKWYG-NH₂ with X2 | 55% | 95% | 73% |

Purity and recoveries of various peptides after application of the purification process according to the invention

### Example 2: purification of naturally occurring and research peptides (P3, P4, P5 and P6)

In a second set five peptides were synthesized. These were H-YFTGSEVENVSVNVH-NH₂ (SEQ ID NO: 3) (P3) a fragment 81-95 of the human cytomegalovirus lower matrix phosphoprotein (CMV), H-PSNPFYEALST-NH₂ (SEQ ID NO: 4) (P4) fragment 510-520 of humane Lemur Tyrosine Kinase 3 (LMTK3), H-DAEFRHDSGYEVHHQKLVFF-NH₂ (SEQ ID NO: 5) (P5) fragment 1-20 of humane amyloid beta and H-CKADEVSMHKWYG-NH₂ (SEQ ID NO: 6) (P6) a peptide sequence intended for research.

The peptide sequences P3, P4, P5 and P6 were synthesized in 100 µmol scale under standard solid phase peptide synthesis conditions, whereby the synthetic resin was treated with acetic anhydride and pyridine after each amino-acid coupling to block unreacted amino groups. Linker X1 was coupled to P3, P4, P5 and P6 on resin by usage of 4 eq. linker X1 (301 mg), 6 eq. oxyma (86 mg) and 6 eq. diisopropylamine (DIEA, 105 µL) for 2 h in 1.3 mL DMF. Thereafter, peptides were cleaved of the synthetic resin by a mixture of TFA / PhOH / PhSH / H₂O / ethanedithiol (EDT, 82.5:5:5:2.5) and precipitated in cold diethylether. The crude peptide mixture was dissolved in 4.5 mL Dimethylsulfoxide (DMSO). Aldehyde modified agarose beads (1.5 mL settled beads) were washed each 3x with water and 0.1 M NaCitrate buffer at pH 4.5. To the DMSO solutions of the peptides 10 vol.% (500 µL) of NaCitrate buffer with 8 M guanidium hydrochloride was added. Then the solution was applied to the agarose beads for 90 minutes, what immobilized the desired peptides quantitatively on the agarose beads. Subsequently a 1 w% solution of L-cysteine in 0.1 M NaCitrate buffer at pH 4.5 was added directly to the immobilisation mixture for 15 min to block unreacted aldehyde groups. Afterwards, the purification media was washed each 3x with DMSO, 6 M guanidium hydrochloride, EtOH/water (7:3) with 0.1 M NaCl, water and MeCN to remove any acetylated termination sequences and other impurities. The cleavage of the immobilized linker was carried out by treating the agarose resin with 10 mL of 50 mg per mL PPh₃ MeCN/AcOH/H₂O (90:5:5). Afterwards the support was rinsed 3x with MeCN/H₂O (9:1) and 2 mL of a solution of H₂O/TFA (60:40) was added for 60 min. Thereafter, to the supernatant 2 mL TFA was added and the resulting mixture was filtered into a centrifuge tube and the support was rinsed 2x with TFA/H₂O (95:5) into the same tube. Et₂O was added 5-fold relative to the TFA-water amount to initiate precipitation and the peptide was gained by centrifugation of the tube and disposal of the organic supernatant.

UPLC-MS was used to verify the purity of the individual phases. The UPLC-chromatograms of the non-purified (without linker molecule) and purified peptides are shown in Figure 4. Identities of the peptides were confirmed by ESI-MS. The yielded peptide amounts after lyophilisation, the calculated recoveries and UV-purities before and after purification are given in Table 2.

**Table 2**

| No. | sequence and linker | UV-purity before | UV-purity after method | weight and recovery |
|---|---|---|---|---|
| P3 | H-YFTGSEVENVSVNVH-NH₂ | 56% | 81% | 36 mg, 74% |
| P4 | H-PSNPFYEALST-NH₂ with X1 | 78% | 91% | 79 mg, 76% |
| P5 | H-DAEFRHDSGYEVHHQKLVFF-NH₂ with X1 | 60% | 87% | 75 mg, 66% |
| P6 | H-CKADEVSMHKWYG-NH₂ with X1 | 60% | 74% | 57 mg, 66% |

Purity and recoveries of various peptides after application of the purification process according to the invention.

### Synthetic steps for the synthesis of 2-((2-(2-bis-(tert-Butoxycarbonyl)-(aminooxy)acetamido) ethyl)carbamoyl)-4-azido-3-bromobenzyl (4-nitrophenyl) carbonate (X1)

### 6-Amino-7-bromophthalide

To a cooled solution (0 °C) of 6-aminophthalide (5.13 g, 34.05 mmol) in THF (80 mL) was added *N*-bromosuccinimide (6.12 g, 34.05 mmol, 1 eq). The cooling bath was removed and the solution stirred for 1 h, then the solvent was removed under reduced pressure. The yellow residue was taken up in ethyl acetate (400 ml) and washed three times with water (200 mL each). The organic phase was dried over magnesium sulfate, filtered and the solvent removed under reduced pressure to give 6-amino-7-bromophthalide as a brown solid (6.53 g, 28.63 mmol, 84%). R_{f} = 0.2 (cyclohexane/ethyl acetate 2:1); UPLC-MS: t_{R} = 1.45 min (gradient 10-90% B in 5 min); UPLC-purity (210 nm) = 83.1%; ESI-MS: (calculated MH⁺: 227.97, 229.96, found: 228.01, 230.01)

### 6-azido-7-bromophthalide

6-Amino-5-bromophthalide (5.54 g, 24.17 mmol) was added to 0 ° C cold hydrochloric acid (1 M, 100 mL). To the cooled suspension was dropwise added conc. sulfuric acid while stirring until the solid was completely dissolved (25 mL), the solution was further cooled until it reached 0 °C again. A solution of sodium nitrite (3.34 g, 48.34 mmol, 2 eq.) in water (17 mL) was added slowly (formation of nitrous gases if solution too warm). After stirring for 10 minutes, a solution of sodium azide (3.14 g, 48.34 mmol, 2 eq). in water (20 mL) was slowly added dropwise (caution: formation of hydrazoic acid). After 30 min, the suspension was extracted with ethyl acetate (200 mL). The aqueous phase was filtered and the filter cake was washed three times with water (100 mL each) and once with cyclohexane (150 mL). 6-azido-7-bromophthalide (6.58 g (94%), 24.17 mmol, quant.) was obtained as a yellow solid. R_{f} = 0.3 (cyclohexane/ethyl acetate 2:1); UPLC-MS: t_{R} = 2.24 min (gradient 10-90% B in 5 min); UPLC-purity (210 nm) = 50.3%

### N-(2-(2-bis-(tert-Butoxycarbonyl)-(aminooxy)acetamido)ethyl)-3-azido-2-bromo-6-(hydroxymethyl)benzamide

6-Azido-7-bromophthalide (5.54 g (94%), 24.17 mmol) was taken up in acetonitrile (150 mL) and the suspension heated to 50 ° C with stirring. Ethylenediamine (23.4 mL, 350.47 mmol, 14.5 eq.) was added, thus solid completely dissolved after 10 minutes. After 1 h stirring at 50 °C, the solvent and excess ethylenediamine were removed under reduced pressure to give a red oil as a residue (8.49 g). Saturated brine (80 mL) was added, the resulting suspension was sonicated for 30 min, stirred at 40 °C for 30 min, and filtered. The filter cake was washed once with saturated brine (50 mL) and once with cyclohexane (100 mL). After drying the filter cake, the title compound was obtained as a yellow solid (3.84 g, 12.2 mmol, 50.6%). Product 3 was also obtained from the filtrate by extraction with ethyl acetate (six times with 150 ml each time) (4.79 g, 15.22 mmol, 63.1%). R_{f} = 0.1 (DCM/MeOH 8:2); UPLC-MS: t_{R} = 1.03 min (gradient 10-90% B in 5 min); UPLC-purity (210 nm) = 83.5%; ESI-MS: (calculated MNa⁺: 336.01, 338.01 g/mol, found: 335.95, 337.96 m/z).

### N-(2-(2-bis-(tert-Butoxycarbonyl)-(aminooxy)acetamido)ethyl)-3-azido-2-bromo-6-(hydroxymethyl)benzamide

To a stirred solution of *bis-(tert-butoxycarbonyl)*-(aminooxy)acetic acid ((Boc)₂AOAc-OH, 4.71 g, 15.86 mmol, 1.3 eq.) and NHS (1.84 g, 15.86 mmol, 1.3 eq.) in acetonitrile (40 mL) is added dicyclohexylcarbodiimide (DCC, 3.30 g, 15.86 mmol, 1.3 eq.). After stirring for 1 h, the solution is separated from the resulting white precipitate by filtration and the filter cake is washed with acetonitril (40 mL). The filtrate is diluted to 120 mL with acetonitrile. *N*-(2-(2-bis-(*tert*-Butoxycarbonyl)-(aminooxy)acetamido)ethyl)-3-azido-2-bromo-6-(hydroxymethyl)benzamide (3.79 g, 12.06 mmol, 1 eq) is taken up in acetonitrile (30 mL) and the suspension is sonicated for 50 min. The filtrate with (Boc)₂AOAc-NHS is then added to this suspension and the reaction mixture is stirred for 2.5 hours. After removal of the solvent under reduced pressure, ethyl acetate (150 ml) is added to the resulting orange oil (10.47 g) and the suspension is sonicated for 10 minutes and stirred at 50 °C. for 10 minutes. After the suspension was washed, (three times with 80 mL 5 w% NaHCO₃ solution (pH 8), once with 80 mL 2% citric acid solution (pH 4.5) and twice with 80 mL brine), the organic phase was separated and dried with magnesium sulfate and the solvent was removed under reduced pressure, a yellow foam was obtained as a crude product (6.84 g). After drying the crude product under high vacuum, the product was obtained as a yellow solid (6.43 g, 75.89% purity (determined with UV / vis), 8.31 mmol, 68.91% yield). Rf = 0.15 (DCM/MeOH 95:5); UPLC-MS: t_{R} = 2.60 min (10-90% MeCN in 3 min), UPLC-purity (21 nm) = 79.1%, ESI-MS: (calculated MNa⁺: 609.13, 611.13 g/mol, found: 609.03, 611.06 m/z).

### 2-((2-(2-bis-(tert-Butoxycarbonyl)-(aminooxy)acetamido)ethyl)carbamoyl)-4-azido-3-bromobenzyl(4-nitrophenyl)carbonate

*N*-(2-(2-bis-(*tert*-Butoxycarbonyl)-(aminooxy)acetamido)ethyl)-3-azido-2-bromo-6-(hydroxymethyl) benzamide (6.39 g (79%), 8.26 mmol) was dissolved in DCM (20 mL) and cooled to 0 ° C. To the solution was first added anhydrous pyridine (1.00 mL, 12.48 mmol, 1.5 eq.) with stirring and then slowly a solution of p-nitrophenyl chloroformate (2.52 g, 12.48 mmol, 1.5 eq.) in DCM (20 mL). The reaction mixture was warmed to room temperature and stirred for 1 h. Under reduced pressure, the solvent is removed and the resulting orange oil (9.99 g) is dissolved in 150 mL ethyl acetate. The suspension was filtered, and the solvent was removed from the filtrate under reduced pressure to give a yellow foamy solid as a crude product (8.85 g). After purification by column chromatography (silica gel, cyclohexane: ethyl acetate 2:1 to 1:1), the product (3.82 g) was taken up in 100 mL diethyl ether, treated with ultrasound for 10 min, stirred for 30 min at 40 ° C and then overnight at -20 °C stored. The product was filtered and washed with 100 mL -20 °C cold diethyl ether and dried after high vacuum as a pale-yellow solid (2.47 g, 3.29 mmol, 39.5%).

R_{f} = 0.25 (Ethyl acetate / Cyclohexane 2:1), UPLC-MS: t_{R} = 3.18 min (10-90% MeCN in 5 min), UPLC-purity (278 nm) = 88.4%, ESI-MS: (calculated MNa⁺: 774.13, 776.13 g/mol, found: 773.91, 775.88 m/z).

### Synthetic steps for the synthesis of 2-((2-(2-bis-(tert-Butoxycarbonyl)-(aminooxy)acetamido) ethyl)carbamoyl)-4-azido-3,5-dibromobenzyl(4-nitrophenyl)carbonate (X2)

### 5,7-Dibromo-6-aminophthalide

6-Aminophthalide (20.00 g, 132.75 mmol) was provided in a 1 L round bottom flask with a stirring bar and 550 mL THF and 30 mL MeCN was added at 0°C. To the solution N-bromosuccinimide was added slowly as a solid through a powder funnel after witch the solution turned brownish. The ice-bath was removed then after some time the solution turned yellow. After 2h stirring at room-temperature UPLC-MS and TLC indicated complete conversion to the dibromide. The solvent was removed under reduced pressure at a rotational evaporator. The remaining solid was dissolved in 600 ml Ethyl acetate and washed 3 times with water. The organic phase was dried with MgSO₄ and after evaporation 39.86 g (129.86 mmol, 98%) of the desired product was gained as a pale yellow solid. R_{f} = 0.6 (cyclohexanes/Ethyl acetate 2:1), UPLC-MS: t_{R} = 2.36 min (10-90% MeCN in 3 min), UPLC-purity (254 nm) = 87.0%, ESI-MS: (calculated MH⁺: 307.95 g/mol, found: 307.76 m/z).

### 5,7-Dibromo-6-azidophthalide

5,7-Dibromo-6-aminophthalide (38.50 g, 124.18 mmol) was dissolved in 200 mL concentrated H₂SO₄ in a 2 L flask, the brown solution was cooled with a big ice bucket then it was slowly added 235 mL 1 M HCI, a precipitate formed during HCI addtion. NaNO₂ (17.31 g, 248.35 mmol, 2 eq) was dissolved in 32 mL water and was added slowly to the suspension after it reached 5 °C. The precipitate dissolved thereafter. The solution was further stirred for 15 min at 0°C and subsequently, NaN₃ (16.31 g, 248.35 mmol, 2 eq) in 75 mL water was added dropwise by Pasteur pipette. Strong formation of gases (N₂, HN₃) was observed. The foamy solution was stirred for 1 h, thereafter 500 ml water was added under cooling with ice.

After foam-building had ceased and solution had reached room-temperature the suspension was filtered by the use of a Büchner funnel, while 2 L of water was used to transfer the solid into the funnel and thereby wash the solid. After drying the wet product in a crystallizing dish under reduced pressure, the product was yielded as a slightly brownish solid (36.01 g, 108.16 mmol, 87.1%). R_{f} = 0.45 (cyclohexanes/Ethyl acetate 2:1), UPLC-MS: t_{R} = 2.89 min (10-90% MeCN in 3 min), UPLC-purity (254 nm) = 95.3%,

### N-(2-aminoethyl)-3-azido-2,4-dibromo-6-(hydroxymethyl)benzamide

5,7-Dibromo-6-azidophtalide (18.00 g, 53.52 mmol) was dissolved in Ethyl acetate (490 mL) insoluble impurities were filtered off and ethylenediamine (52.73 mL, 749.32 mmol, 14 eq) was added at 0 °C. The reaction was stirred at room temperature for 1 h, after which UPLC-MS showed quantitative conversion. The reaction mixture was transferred into a separation funnel to which 100 mL Brine was added. After separation of the aqueous phase the organic phase was dried with MgSO₄ and the desired product was gained after evaporating the organic solvent on rotational evaporator as an orange solid. (20.50 g, 52.16 mmol, 97.4%). R_{f} = 0.25 (DCM/MeOH 8:2), UPLC-MS: t_{R} = 1.80 min (10-90% MeCN in 3 min), UPLC-purity (254 nm) = 84.2%, ESI-MS: (calculated MH⁺: 393.93 g/mol, found: 393.87 m/z).

### N-(2-(2-bis-(tert-Butoxycarbonyl)-(aminooxy)acetamido)ethyl)-3-azido-2,4-dibromo-6-(hydroxymethyl)benzamide

*Bis-(tert-Butoxycarbonyl)-(aminooxy)acetic* acid ((Boc)₂AOAcOH, 17.30 g, 58.18 mmol, 1.1 eq.) and *N*-hydroxysuccinimide (NHS, 6.76 g, 58.18 mmol, 1.1 eq.) was dissolved in 350 mL Acetonitril. To this solution was added dicyclohexylcarbodiimide (DCC, 12.13 g, 58.18 mmol, 1.1 eq.) as a solid, after dissolution of DCC a white precipitate formed. The reaction mixture was stirred for 1 h at room temperature where the (Boc)₂AOAc-NHS ester was quantiatively formed according to UPLC-MS. Thereafter the mixture was filtered into a 1 L flask to remove the DCC-urea. *N*-(2-aminoethyl)-3-azido-2,4-dibromo-6-(hydroxymethyl)benzamide (20.5 g, 52.90 mmol) was dissolved in 530 mL Ethyl acetate and subsequently added to the solution of (Boc)₂AOAc-NHS. The mixture was stirred at room temperature for 1 h after which the completion of the reaction was confirmed by TLC and UPLC-MS. Additional formed precipitate was filtered off and the organic phase was washed 2x with 5% NaHCO₃ (each 200 mL), 1x brine and 2x 2% citric acid solution(pH 4.5) / brine 1:1 (each 150 mL). The organic phase was dried with MgSO₄ and hence, the organic solvent was removed *in vacuo* and the title compound was obtained as a pale yellow oil (37.70 g, 56.58 mmol, quantitativ). R_{f} = 0.4 (DCM/MeOH 95:5), UPLC-MS: t_{R} = 2.97 min (10-90% MeCN in 3 min), UPLC-purity (254 nm) = 54.2%, ESI-MS: (calculated MH⁺: 667.05, MNa⁺: 689.04 g/mol, found: 688.95 m/z).

### 2-((2-(2-bis-(tert-Butoxycarbonyl)-(aminooxy)acetamido)ethyl)carbamoyl)-4-azido-3,5-dibromobenzyl (4-nitrophenyl) carbonate

N-(2-(2-bis-(tert-Butoxycarbonyl)-(aminooxy)acetamido)ethyl)-3-azido-2,4-dibromo-6-(hydroxymethyl)benzamide (37.70 g (94%), 52.90 mmol was provided in CH₂Cl₂ (170 mL) and (dry, stored over molecular sieve) pyridine (4.72 mL, 58.50 mmol, 1.1 eq) was added. Thereafter, 4-nitrophenylchloroformiate (12.03 g, 58.50 mmol, 1.1 eq) was added slowly as a solid at room temperature keeping temperature constant by usage of a water bath. Reaction may cause DCM to evaporate at the center of the flask. Indicated by LCMS and TLC indicated a complete reaction after 1 h. Dichloromethane was removed *in vacuo* yielding 52 g crude brown oil and the residue was dissolved in 500 mL ethyl acetate and washed 2x 2% citric acid solution (pH 4.5) / brine 1:1 (each 250 mL) and 1 x brine 150 mL. The organic phase was dried by usage of MgSO₄. Afterwards this suspension was filtered over a 50 g silica plug in a glas frit, whereas the orange and reddish impurities remained on silica. The organic solvent was removed from the filtrate under reduced pressure till a highly viscous slightly amber oil remained. To this oil 70 ml of Et₂O was added and the two-phasic emulsion was turned on a rotational evaporator at 45 °C for 10 min till one homogeneous phase was formed. A small sand corn was added to the flask as a crystallization initiator and the flask was put in a refrigerator overnight (16 h). In the flask a sluggish precipitate had formed. Further 200 mL of cold (-25 °C) ether was added to the flask and the flask was gently shaken and stirred in an ice-bath. The white star-like crystals were transferred and washed with additional 200 mL cold Et₂O into a paper filter filled Büchner-funnel. Thus, yielding the title compound as a white solid (29.95 g, 36.02 mmol, 68.1%). R_{f} = 0.6 (Ethyl acetate / Cyclohexane 2:1), UPLC-MS: t_{R} = 2.86 min (30-95% MeCN in 3 min), UPLC-purity (278 nm) = 93.5%, ESI-MS: (calculated MH⁺: 832.06, MNa⁺: 854.04 g/mol, found: 853.87 m/z).

## Claims

1. A compound of formula 1, X-T_{b}-Vₐ-U-Y-Z (1), wherein
- X is selected from a moiety of formula 2, 2a, 3, 3a or 4, in particular of formula 2, 2a, 3 or 3a, more particularly of formula 2 or 2a, 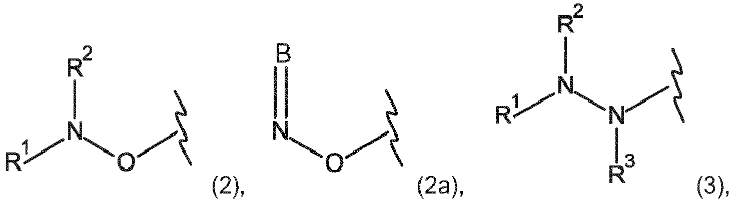 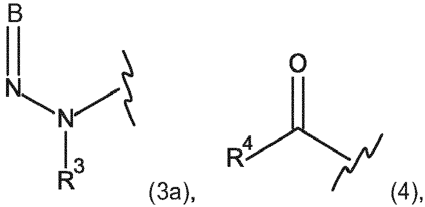 wherein
- each R¹ and R² is independently from each other selected from H or B, wherein at least R¹ or R² is B,
- R³ is selected from H or B,
- R⁴ is selected from H, C₁-C₁₂-alkyl or aryl, wherein the aldehyde or keto group may be protected by an acid labile protecting group,
- B is an acid labile amine protecting group,
- T is a linear or branched spacer comprising at least one of the moieties -C₁₋₁₂-alkyl-, (-C₂H₄O-)₁₋₁₂, -C(=O)-, -C(=O)-JR⁹-, -JR⁹-C(=O)-, -JR⁹-, wherein
J is C or N, in particular N,
in particular T is a spacer selected from
-C₁-C₁₂-alkyl-,in particular C₁₋₆-alkyl, more particularly C₁₋₃-alkyl, -R⁵-C(=O)-,-R⁵-C(=O)-NR⁹-R⁶-, -R⁵-C(=O)-NR⁹-, -C(=O)-NR⁹-R⁶-, -R⁵⁻NR⁹-C(=O)-R⁶-, -R⁵-NR⁹-R^{5'}-NR^{9'}C(=O)-R⁶-, -R⁵-C(=O)-NR⁹-R^{5'}-NR^{9'}-C(=O)-R⁶-^{,} -R⁵-NR⁹-, -R⁵-NR⁹-R⁶-, -R⁵-NR⁹-R^{5'}-NR^{9'}-R⁶-, -R⁵-C(=O)-NR⁹-R^{5'}-NR^{9'}-R⁶-, -R⁵-C(=O)-O-R⁶-,-C(=O)-O-R⁶-, -R⁵-phenyl-R⁶-, -R⁵-phenyl-, -phenyl-R⁶-, -phenyl-, wherein
R⁵, R^{5'} and R⁶ are independently from each other selected from C₁-C₁₂-alkyl or (-C₂H₄O-)₁₋₁₂, in particular C₁-C₆ alkyl, particularly C₁-C₃ alkyl, and wherein
R⁹ and R^{9'} are independently from each other selected from H, C₁₋₄-alkyl, -C₁₋₆-alkyl-NH₂, -C₁₋₆-alkyl-NHB, -C₁₋₆-alkyl-NB₂, -R¹⁵, -C₁₋₆-alkyl-R¹⁵, -C₁₋₆-alkyl-NH-R¹⁵, in particular from H and C₁₋₂alkyl, more particularly R⁹ is H, wherein
B is an independently selected acid labile amine protecting group,
R¹⁵ is a blocking agent that is able to react with an aldehyde moiety, in particular R¹⁵ is selected from cysteinyl, threoninyl, 2-mercaptoethanol, cysteamine, ethandithiole, hydroxylamine, O-methylhydroxylamine, N-methylhydroxylamine, dithiothreitol, hydrazine, in particular cysteinyl and N-methylhydroxylamine, more particularly cysteinyl, wherein amine and/or thiol moieties of the blocking agent may be protected by an independently selected acid labile amine protecting group B, particularly Boc, and/or an acid labile thiol protecting group, particularly trityl,
- b is 0 or 1, in particular 1,
- V is an electron-withdrawing moiety selected from -NR¹¹-C(=O)-, -C(=O)-NR¹¹-, - S(=O)-, -NR¹²-, -piperazinyl-, -pyridinyl, pyrimidinyl, pyrazinyl, pyridazinyl, 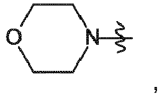 -C(=O)-, -C(=O)-O-, in particular -NR¹¹-C(=O)-, -C(=O)-NR¹¹-, -S(=O)-, -NR¹²-, -piperazinyl-, -pyridinyl-, pyrimidinyl, more particularly from -NH-C(=O)-, -C(=O)-NH-, -N-(CH₃)-, -piperazinyl-, -pyridinyl-, pyrimidinyl, wherein
R¹¹ is selected from H and C₁₋₄-alkyl, in particular from H and C₁₋₂alkyl, more particularly R¹¹ is H,
R¹² is selected from H and C₁₋₄-alkyl, in particular from H and C₁₋₂alkyl, more particularly R¹² is methyl,
- a is 0 or 1, wherein the sum of a and b is 1 or 2,
- U is a phenyl or a five- or six-membered heteroaryl moiety, in particular a phenyl or a six-membered heteroaryl moiety, more particularly a phenyl, that is bound to at least one of the moieties V, W_{q} and Eₙ and that may optionally be substituted by C₁₋₆-alkyl, in particular C₁₋₃-alkyl, wherein
V is defined as described above,
W is selected from -N₃, -S(=O)-R⁸, -S-S-R⁸, -O-CH₂-N₃, -O-C(=O)-O-CH₂-N₃,-N=N-phenyl, -N=N-R⁸, 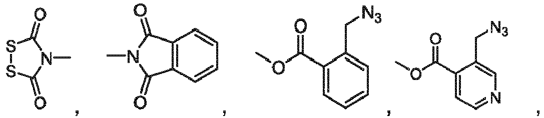 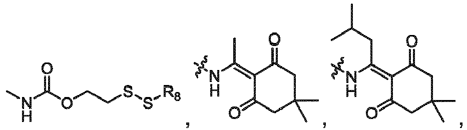 in particular -N₃, -N=N-R⁸, -O-CH₂-N₃, -S-S-R⁸, wherein R⁸ is pyridyl, pyrimidinyl, pyrazinyl, pyridazyl, -C₁C₆-alkyl or -(CH₂)ₚ-NMe₂, in particular pyridyl or -C₁-C₆-alkyl, with p being 1, 2, 3 or 4,
E is an electron withdrawing group under acidic conditions,
n being is an integer between 0 and 4, in particular 0 and 2, more particularly 0 or 1, and q is an integer between 0 and 4, in particular 0 and 2, more particularly 0 and 1, wherein the sum of n and q is equal or lower than 4, and wherein
in case of U being a phenyl moiety and Y being -(CH₂)ₘ-O-C(=O)-, the sum of Hammett constants of V, W, E under acidic conditions is larger than 0.45,
- Y is -(CH₂)ₘ-C(=O)- or -(CH₂)ₘ-O-C(=O)- with m being 1, 2 or 3, in particular 1 or 2, more particularly 1,
- Z is an electron-withdrawing leaving group.

2. The compound of claim 1, wherein E is selected from pyridyl, pyrimidinyl, pyrazinyl, pyridazyl, ,-N=N-phenyl, -N=N-R⁸, -F, -Cl, -Br, -I, -CN, -NO₂, -N₃, -CF₃, -SO₃H, -CO₂H, - C(=O)NH₂, -SO₂Me, -SOMe, -SO₂Et, -SOEt, in particular pyridyl, pyrimidinyl, pyrazinyl, pyridazinyl, ,-N=N-phenyl, -N=N-R⁸, -F, -Cl, -Br, -I, -CN, -NO₂, -N₃, -CF₃, -SO₃H, -CO₂H, more particularly pyridyl, pyrimidinyl, pyridazinyl or -Br with
R⁸ being pyridyl, pyrimidinyl, pyrazinyl, pyridazyl, -C₁-C₆-alkyl or -(CH₂)ₚ-NMe₂, in particular pyridyl or -C₁-C₆-alkyl, with p being 1, 2, 3 or 4.

3. The compound according to any one of the preceding claims, wherein
- B is selected from Boc (-C(=O)OtBu), Eei (=CMeOEt, 1-ethoxyethylidene) trityl (-C(Ph)₃), Mmt (-C(Ph)₂C₆H₄OMe), DMT (-C(Ph)(C₆H₄OMe)₂), Cbz (-C(=O)OCH₂Ph), benzylideneamine (=CPh), phtalimides (=(CO)₂C₆H₄), p-toluenesulfonamides (-SO₂C₆H₄Me), benzylamine (-CH₂Ph), acetamides (-COMe), trifluoroacetamide (-COCF₃), Dde (1-(4,4-dimethyl-2,6-dioxocyclohex-1-ylidene)-3-ethyl) and 1-(4,4-dimethyl-2,6-dioxocyclohex-1-ylidene)-3-methylbutyl (ivDde), wherein particularly B is Boc or Eei, wherein more particularly B is Boc, and/or the
- acetal- or ketal protecting groups are selected from 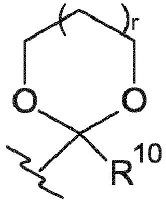 or 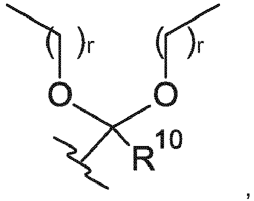
wherein r is 0 to 12, in particular 0 to 6, more particularly 0, 1 or 2, and
R¹⁰ is -C₁-C₁₂-alkyl-, in particular C₁₋₆-alkyl, more particularly C₁₋₃alkyl.

4. The compound according to any one of the preceding claims, wherein T is selected from
-C₁-C₁₂-alkyl-, in particular C₁₋₆-alkyl, more particularly C₁₋₃-alkyl, -R⁵-C(=O)-, -R⁵-C(=O)-NR⁹-, -R⁵-NR⁹-C(=O)-R⁶-, -R⁵-C(=O)-NR⁹-R⁶-, -R⁵-C(=O)-NR⁹-R^{5'}-NR^{9'}C(=O)-R⁶-, in particular C₁₋₃-alkyl, -R⁵-C(=O)-NR⁹-, -R⁵-NR⁹-C(=O)-R⁶-, more particularly C₁₋₃-alkyl or - R⁵-C(=O)-NR⁹-, with R⁵, R^{5*}, R⁶, R^{9'} and R⁹ being as defined above.

5. The compound according to any one of the preceding claims, wherein the five- or six-membered heteroaryl moiety of U comprises 1 or 2 heteroatoms, in particular the five-membered heteroaryl moiety of the moiety U is selected from pyrazole, imidazole, and the six-membered heteroaryl moiety of the moiety U is selected from pyridine, pyridazine, pyrimidine, pyrazine, particularly pyridine.

6. The compound according to any one of the preceding claims, wherein U is selected from a moiety of formula 5, 6, 7 or 8, in particular of formula 5 or 6, 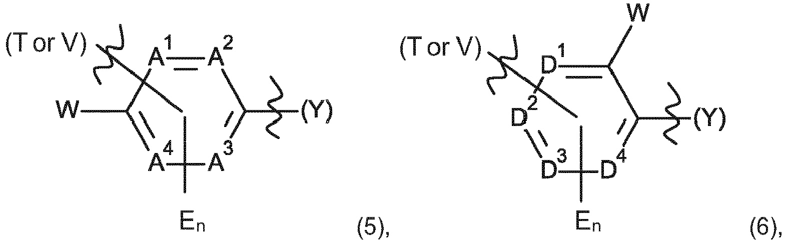 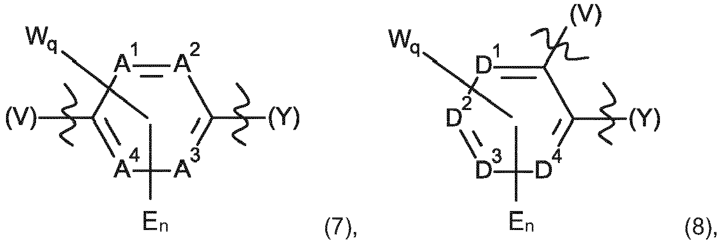 wherein
T, V, Y, W and E are defined as described above,
in case of formula 5 and 6, U is bound to the moiety T or V,
in case of formula 7 and 8, U is bound to the moiety V,
A¹, A², A³, A⁴ and D¹, D², D³, D⁴ are independently from each other selected from C, N, S and O, in particular from C and N, wherein 2 to 4 moieties of A¹, A², A³ and A⁴ or of D¹, D², D³ and D⁴ are C, particularly 3 or 4 moieties of A¹, A², A³ and A⁴ or of D¹, D², D³ and D⁴ are C, more particularly all moieties A¹, A², A³ and A⁴ or of D¹, D², D³ and D⁴ are C,
n is
in case of formulas 5 and 6 an integer between 0 and 3, in particular 0 and 2, in case of formulas 7 and 8 an integer between 0 and 4, in particular 0 and 2, more particular 0 and 1,
q is an integer between 0 and 4, in particular 0 and 2, more particular 0 and 1, wherein the sum of n and q is equal or lower than 4.

7. The compound according to claim 5, wherein U is selected from a moiety of formula 9, 10, 11 or 12, in particular of formula 9 or 10, 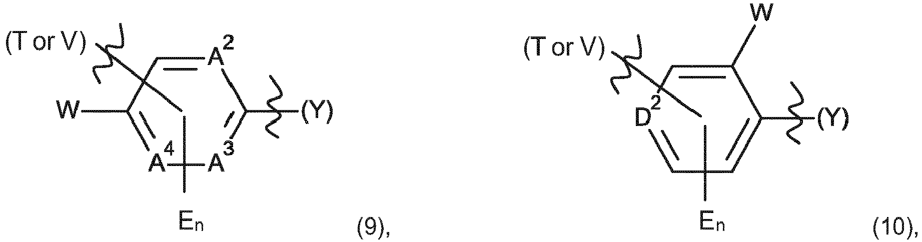 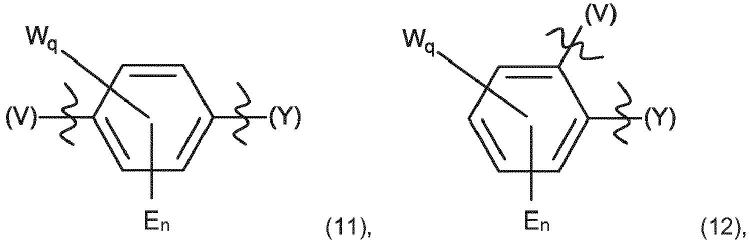 wherein
T, V, Y, W, E, q and n are defined as described above,
in case of formula 9 and 10, U is bound to the moiety T or V,
in case of formula 11 and 12, U is bound to the moiety V,
all moieties A², A³ and A⁴ are C or two of A², A³ and A⁴ are C and the other two of A², A³ and A⁴ is N, in particular A² and A³ are both C, and
D² is C or N, in particular C.

8. The compound according to any one of the preceding claims, wherein U is selected from a moiety of formula 13, 14, 15, 16, 17,18, 19, 20 or 21, in particular of formula 13 to 19, more particular of formula 15 or 19, 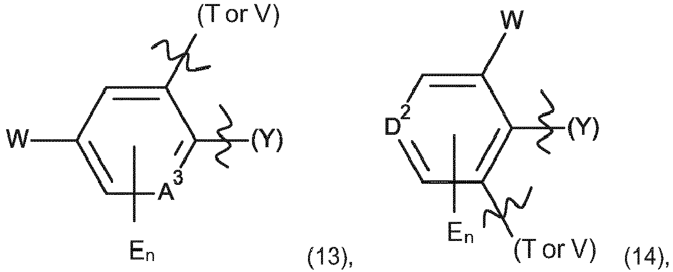 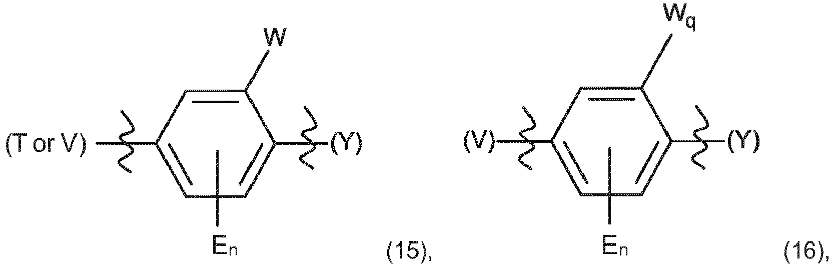 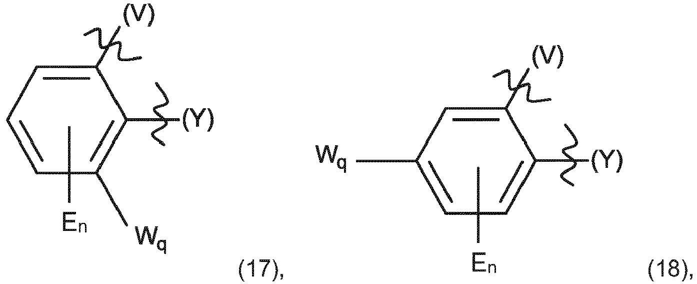 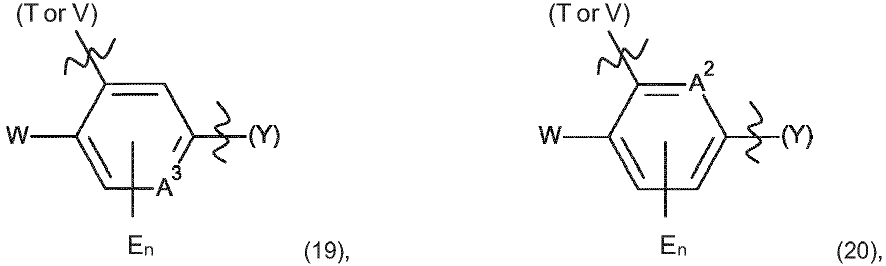 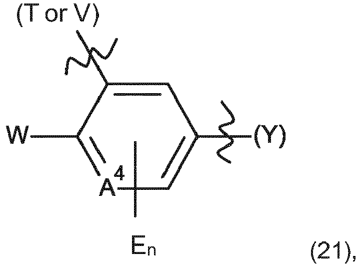 wherein
T, V, Y, W, E, q and n are defined as described above,
in case of formula 13, 14 and 15, U is bound to the moiety T or V,
in case of formula 16, 17 and 18, U is bound to the moiety V,
A², A³ and A⁴ is C or N, in particular C,
D² is C or N.

9. The compound according to any one of claims 1 to 3, wherein Z is selected from: -F, - Cl, -Br, -I, -N₃, -OH, -O(C=O)CH₂(C=O)OH, -SR¹⁴, -OCF₃, -OCH₂CF₃, -OSO₂CF₃, - SO₂C₆H₄CH₃, -SO₂CF₃, -SO₂CH₃ 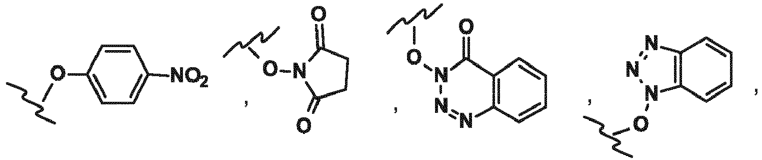 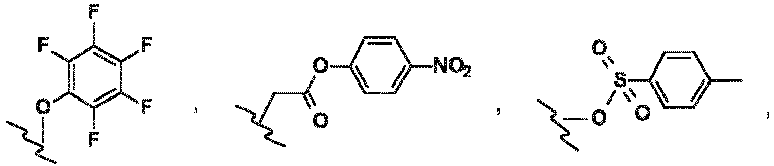 in particular -OH, -Cl, 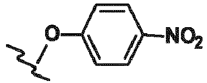 or 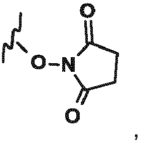 in particular -OH, 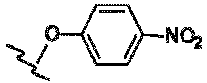 or 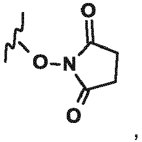 wherein R¹⁴ is an C₁-C₆-alkyl-, an arylic- or benzylic substituent.

10. A method for purifying peptides comprising the steps of
- providing a crude linker-modified peptide, wherein the crude peptide is covalently bound to a linker molecule according to claim 1,
- in a coupling step, coupling the linker-modified peptide with a solid support yielding an immobilized linker-modified peptide,
- in a releasing step, releasing the peptide, in particular by adding a reducing agent under acidic conditions.

11. The method according to claim 10, wherein in the releasing step, a reduced intermediate **characterized by** a reduced linker moiety of the immobilized linker-modified peptide is achieved and the peptide is released from said reduced intermediate by a trigger, in particular a change in temperature and/or pH, more particularly by increasing the pH to pH > pKa of the most basic heteroatom of the linker moiety of the reduced intermediate.

12. The method according to any one of claims 10 to 11, wherein the linker-modified peptide is additionally bound to a synthesis resin and the synthesis resin is cleaved off before step (b) is performed.

13. The method according to any one of claims 10 to 12, wherein the linker molecule according to claim 1 comprises a moiety W and/or E that comprises an azide moiety.

14. The method according to any one of claims 10 to 13, wherein non-reacted aldehyde moieties of the solid support are blocked using a blocking agent after performing step (b), in particular by using a blocking agent selected from cysteine, threonine, 2-mercaptoethanol, cysteamine, ethandithiole, hydroxylamine, O-methylhydroxylamine, N-methylhydroxylamine, dithiothreitol, hydrazine, more particularly a blocking agent selected from cysteine and N-methylhydroxylamine.

15. The method according to any one of claims 10 to 14, wherein the reducing agent is selected from triphenylphosphine, trimethylphosphine, triethylphosphine, tributylphosphine or tris(2-carboxyethyl)phosphine, trimethyl phosphite, triethyl phosphite, tributyl phosphine, diethyl phosphite, 5,5'-Dithiobis(2-nitrobenzoic acid), sodium dithionite (Na₂S₂O₄), ethandithiole, Propandithiol, dithiothreitol, Na₂S, NaSH, glutathione, 2,2'-dithiodipyridine, BH₃, 4,4,5,5-tetramethyl-1,3,2-dioxaborolane, catechol borane, borane tetrahydrofuran, borane dimethyl sulfide, borane dimethylamine complex, borane triphenylphosphine complex, borane tert-butylamine, LiAlH₄, LiBH₄, NaBH₄, NaBH₃CN, NaBH(OMe)₃, NaBH(OCCH₃)₃, LiAlH(OCMe₃)₃, hydroquinone, sodium ascorbate, ascorbic acid, hydrazine, NH=NH, formaldehyde, in particular triphenylphosphine, tributylphosphine, trimethylphosphine, or tris(2-carboxyethyl)phosphine, sodium dithionite (Na₂S₂O₄), borane dimethyl sulfide, borane triphenylphosphine complex, NaBH₄, ascorbic acid, more particularly triphenylphosphine and trimethylphosphine.
